(19) Europäisches Patentamt / European Patent Office / Office européen des brevets

(11) **EP 2 951 574 B1**

(12) **EUROPEAN PATENT SPECIFICATION**

(45) Date of publication and mention of the grant of the patent:
**27.03.2019  Bulletin 2019/13**

(21) Application number: **14746042.2**

(22) Date of filing: **03.02.2014**

(51) Int Cl.:
*G01N 33/48* (2006.01)

(86) International application number:
**PCT/US2014/014466**

(87) International publication number:
**WO 2014/121205 (07.08.2014 Gazette 2014/32)**

(54) **MEASURING EMBRYO DEVELOPMENT AND IMPLANTATION POTENTIAL WITH TIMING AND FIRST CYTOKINESIS PHENOTYPE PARAMETERS**

MESSUNG DER ENTWICKLUNG EINES EMBRYOS SOWIE EINES IMPLANTATIONSPOTENZIALS ANHAND VON ZEIT UND DER ERSTEN CYTOKINESE-PHÄNOTYP-PARAMETER

MESURE DU POTENTIEL DE DÉVELOPPEMENT ET D'IMPLANTATION D'UN EMBRYON, FAISANT APPEL À DES PARAMÈTRES DE DURÉE ET DE PHÉNOTYPE RÉSULTANT DE LA PREMIÈRE CYTOKINÈSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.02.2013  US 201361759607 P**
**14.03.2013  US 201361783988 P**
**01.05.2013  US 201361818127 P**

(43) Date of publication of application:
**09.12.2015  Bulletin 2015/50**

(73) Proprietor: **Progyny, Inc.**
**Menlo Park CA 94025 (US)**

(72) Inventors:
• **SHEN, Shehua**
**Menolo Park, CA 94025 (US)**
• **CHEN KIM, Alice, A.**
**Menolo Park, CA 94025 (US)**
• **WIRKA, Kelly, Athayde**
**Menolo Park, CA 94025 (US)**
• **SURAJ, Vaishali**
**Menolo Park, CA 94025 (US)**
• **TAN, Lei**
**Menolo Park, CA 94025 (US)**

(74) Representative: **CMS Cameron McKenna Nabarro Olswang LLP**
**Cannon Place**
**78 Cannon Street**
**London EC4N 6AF (GB)**

(56) References cited:
WO-A1-2012/116185    WO-A1-2012/116185
WO-A2-2012/047678    US-A1- 2011 092 762

• VICTORIA BURRUEL ET AL: "Abnormal Early Cleavage Events Predict Early Embryo Demise: Sperm Oxidative Stress and Early Abnormal Cleavage", SCIENTIFIC REPORTS, vol. 4, 13 October 2014 (2014-10-13), page 6598, XP055234662, DOI: 10.1038/srep06598
• CHAVEZ ET AL.: 'Dynamic blastomere behaviour reflects human embryo ploidy by the four-cell stage.' NATURE COMMUNICATIONS 04 December 2012, pages 1 - 12, XP055169858

**Description**

CROSS REFERENCE TO RELATED APPLICATIONS

This application claims priority to U.S. Provisional Appln. No. 61/759,607 filed February 1, 2013; U.S. Provisional Appln. No. 61/783,988, filed March 14, 2013; and U.S. Provisional Appln. No. 61/818,127, filed May 1, 2013, each of which is herein incorporated by reference in its entirety.

FIELD OF THE INVENTION

This invention relates to the field of biological and clinical testing, and particularly the imaging and evaluation of zygotes/embryos from both humans and animals.

BACKGROUND OF THE INVENTION

Infertility is a common health problem that affects 10-15% of couples of reproductive-age. In the United States alone in the year 2006, approximately 140,000 cycles of in vitro fertilization (IVF) were performed (cdc.gov/art). This resulted in the culture of more than a million embryos annually with variable, and often ill-defined, potential for implantation and development to term. The live birth rate, per cycle, following IVF was just 29%, while on average 30% of live births resulted in multiple gestations (cdc.gov/art). Multiple gestations have well-documented adverse outcomes for both the mother and fetuses, such as miscarriage, pre-term birth, and low birth rate. Potential causes for failure of IVF are diverse; however, since the introduction of IVF in 1978, one of the major challenges has been to identify the embryos that are most suitable for transfer and most likely to result in term pregnancy.

The understanding in the art of basic embryo development is limited as studies on human embryo biology remain challenging and often exempt from research funding. Consequently, most of the current knowledge of embryo development derives from studies of model organisms. Embryos from different species go through similar developmental stages, however, the timing varies by species. These differences and many others make it inappropriate to directly extrapolate from one species to another. (Taft, R.E. (2008) Theriogenology 69(1):10-16). The general pathways of human development, as well as the fundamental underlying molecular determinants, are unique to human embryo development. For example, in mice, embryonic transcription is activated approximately 12 hours post-fertilization, concurrent with the first cleavage division, whereas in humans embryonic gene activation (EGA) occurs on day 3, around the 8-cell stage (Bell, C. E., et al. (2008) Mol. Hum. Reprod. 14:691-701; Braude, P., et al. (1988) Nature 332:459-461; Hamatani, T. et al. (2004) Proc. Natl. Acad. Sci. 101:10326-10331; Dobson, T. et al. (2004) Human Molecular Genetics 13(14):1461-1470). In addition, the genes that are modulated in early human development are unique (Dobson, T. et al. (2004) Human Molecular Genetics 13(14):1461- 1470). Moreover, in other species such as the mouse, more than 85% of embryos cultured in vitro reach the blastocyst stage, one of the first major landmarks in mammalian development, whereas cultured human embryos have an average blastocyst formation rate of approximately 30-50%, with a high incidence of mosaicism and aberrant phenotypes, such as fragmentation and developmental arrest (Rienzi, L. et al. (2005) Reprod. Biomed. Online 10:669-681; Alikani, M., et al. (2005) Mol. Hum. Reprod. 11:335-344; Keltz, M. D., et al. (2006) Fertil. Steril. 86:321-324; French, D. B., et al. (2009) Fertil. Steril.). In spite of such differences, the majority of studies of preimplantation embryo development derive from model organisms and are difficult to relate to human embryo development (Zernicka-Goetz, M. (2002) Development 129:815-829; Wang, Q., et al. (2004) Dev Cell. 6:133-144; Bell, C. E., et al. (2008) Mol. Hum. Reprod. 14:691-701; Zemicka-Goetz, M. (2006) Curr. Opin. Genet. Dev. 16:406-412; Mtango, N. R., et al. (2008) Int. Rev. Cell. Mol. Biol. 268:223-290).

Traditionally in IVF clinics, human embryo viability has been assessed by simple morphologic observations such as the presence of uniformly-sized, mononucleate blastomeres and the degree of cellular fragmentation (Rijinders PM, Jansen CAM. (1998) Hum Reprod 13:2869-73; Milki AA, et al. (2002) Fertil Steril 77:1191-5). More recently, additional methods such as extended culture of embryos (to the blastocyst stage at day 5) and analysis of chromosomal status via preimplantation genetic diagnosis (PGD) have also been used to assess embryo quality (Milki A, et al. (2000) Fertil Steril 73:126-9; Fragouli E, (2009) Fertil Steril Jun 21 [EPub ahead of print]; El-Toukhy T, et al. (2009) Hum Reprod 6:20; Vanneste E, et al. (2009) Nat Med 15:577-83). However, potential risks of these methods also exist in that they prolong the culture period and disrupt embryo integrity (Manipalviratn S, et al. (2009) Fertil Steril 91:305-15; Mastenbroek S, et al. (2007) N Engl J Med. 357:9-17).

US Patent Nos. 7,963,906; 8,323,177 and 8,337,387 describe novel timing parameters including the duration of the first cytokinesis, the interval between cytokinesis 1 and cytokinesis 2, the interval between mitosis 1 and mitosis 2, the interval between cytokinesis 2 and cytokinesis 3 and the interval between mitosis 2 and mitosis 3 that are useful in selecting embryos with good developmental potential that are likely to reach the blastocyst stage, implant into the uterus and/or be born live.

Not withstanding the recent developments in time lapse imaging that allow clinicians to select embryos with greater developmental potential based on timing parameters of the first few cell cycles, current embryo selection relies primarily on morphological evaluations which are very subjective and offer limited predictive value of embryo viability. Failure to correctly identify the most viable embryos can lead to unsuccessful IVF treatment or multiple gestation pregnancy. Time-lapse imaging technology allows real time embryo monitoring and provides additional insight into human embryo developmental biology. This technology has allowed for the identification of new atypical embryo phenotypes and new timing parameters that may impact embryo development including the novel syngamy parameters described herein.

SUMMARY OF THE INVENTION

The invention provides for methods, compositions and kits for determining the likelihood that one or more embryos will reach the blastocyst stage become a good quality blastocyst, or implant into the uterus or be born live or be euploid. These methods, compositions and kits are useful in methods of treating infertility in humans and other animals.

In some aspects of the invention, methods are provided for determining the likelihood that an embryo will reach the blastocyst stage and/or become a good quality blastocyst and/or implant into the uterus. In some aspects determining the likelihood of reaching the blastocyst stage and/or becoming a good quality blastocyst and/or implanting into the uterus and/or being euploid is determined by deselecting with high specificity one or more human embryos that is not likely to reach the blastocyst stage, become a good quality blastocyst, implant into the uterus, be born live or be euploid wherein at least about 70%, 75%, 80%, 85%, 90%, 95% or more or 100%) of the human embryos deselected are not likely to reach the blastocyst stage and/or implant into the uterus and/or be born live and/or be euploid. In such aspects, cellular parameters of an embryo are measured to arrive at a cellular parameter measurement which can be employed to provide a determination of the likelihood of the embryo to reach the blastocyst stage and/or implant into a uterus and/or be euploid, which determination may be used to guide a clinical course of action. In some embodiments, the cellular parameter is a morphological event that is measureable by time-lapse microscopy. In certain embodiments, the morphological event includes determination of cell numbers during cleavage events, specifically, determining the number daughter cells produced from a single cleavage event.

In particular embodiments, the morphological event is the duration of P1 or first cytokinesis (i.e. the time period between the appearance of the 1st cleavage furrow to completion of the 1st cell division) and/or one or more P1 phenotypes. In a particular embodiment, embryos having a prolonged P1 or first cytokinesis (i.e. the time period between the appearance of the 1st cleavage furrow to completion of the 1st cell division) ≥0.5 hr are less likely to be euploid, reach the blastocyst stage, develop into a good quality blastocyst and/or implant into the uterus and therefore are deselected. In some embodiment, embryos having a prolonged P1 or first cytokinesis (i.e. the time period between the appearance of the 1st cleavage furrow to completion of the 1st cell division) ≥0.5 hr while also displaying one or more abnormal P1 phenotypes (including, e.g., membrane ruffling, oolemma ruffling with or without formation of one or more pseudo cleavage furrows prior to completing the first cytokinesis (P1)) are less likely to be euploid, reach the blastocyst stage, develop into a good quality blastocyst, implant and/or be born live. These embryos show lower potential of development and may have lower potential to implant into the uterus and therefore are deselected. In some embodiments, embryos displaying one or more abnormal P1 phenotypes (including, e.g., membrane ruffling, oolemma ruffling with or without formation of one or more pseudo cleavage furrows prior to completing the first cytokinesis (P1)) are less likely to be euploid, reach the blastocyst stage, develop into a good quality blastocyst, implant in to the uterus and/or be born live and therefore are deselected. In some embodiments, embryos displaying membrane or oolemma ruffling prior to or during the first cytokinesis (P1) and having a prolonged P1 or first cytokinesis (i.e. the time period between the appearance of the 1st cleavage furrow to completion of the 1st cell division) ≥0.5 hr are less likely to be euploid, reach the blastocyst stage, develop into a good quality blastocyst, implant into the uterus and/or be born live and therefore are deselected. In some embodiments, embryo displaying membrane or oolemma ruffling prior to or during the first cytokinesis (P1) and forming one or more pseudo cleavage furrows prior to completing the first cytokinesis (P1) are less likely to be euploid, reach the blastocyst stage, develop into a good quality blastocyst and/or implant into the uterus and therefore are deselected. In some embodiments, embryos forming one or more pseudo cleavage furrows prior to completing the first cytokinesis (P1) and having a prolonged P1 or first cytokinesis (i.e. the time period between the appearance of the 1st cleavage furrow to completion of the 1st cell division) ≥0.5 hr are less likely to be euploid, reach the blastocyst stage, develop into a good quality blastocyst and/or implant into the uterus and therefore are deselected. In some embodiments, embryos displaying membrane or oolemma ruffling prior to or during the first cytokinesis (P1), forming one or more pseudo cleavage furrows prior to completing the first cytokinesis (P1) and having a prolonged P1 or first cytokinesis (i.e. the time period between the appearance of the 1st cleavage furrow to completion of the 1st cell division) ≥0.5 hr are less likely to be euploid, reach the blastocyst stage, develop into a good quality blastocyst and/or implant into the uterus and therefore are deselected.

In some embodiments, in addition to measuring the duration of P1 or first cytokinesis (i.e. the time period between the appearance of the 1st cleavage furrow to completion of the 1st cell division) and/or one or more P1 phenotypes (e.g. membrane ruffling, oolemma ruffling and/or formation of one or more pseudo cleavage furrows prior to completing the first cytokinesis (P1)), one or more additional cellular parameters are measured including: the duration of a cytokinesis event, e.g. the duration of cytokinesis 1, the time interval between cytokinesis 1 and cytokinesis 2; or the time interval between cytokinesis 2 and cytokinesis 3. In some embodiments, the one or more cellular parameters is: the duration of a mitotic event, e.g. the time interval between mitosis 1 and mitosis 2; and the time interval between mitosis 2 and mitosis 3. In certain embodiments, the duration of cell cycle 1 is also utilized as a cellular parameter. In certain embodiment, the time between fertilization and the 5 cell stage is also utilized as a cellular parameter. In certain embodiments the time period between syngamy and the beginning of the first cytokinesis is measured. In some embodiments, the cell parameter measurement is employed by comparing it to a comparable cell parameter measurement from a reference embryo, and using the result of this comparison to provide a determination of the likelihood of the embryo to be euploid, reach the blastocyst stage and/or become a good quality blastocyst and/or implant into the uterus. In some embodiments, the embryo is a human embryo.

In one embodiment, embryos are monitored to determine their phenotype during syngamy. In further embodiments, embryos are deselected as being less likely to reach the blastocyst stage or develop into good quality blastocysts or implant into the uterus or be born live or be euploid when syngamy is abnormal (AS). In a particular embodiment, an embryo is determined to display AS when there is disordered PN movement, delayed dispersion of nuclear envelopes, active oolema movement before the dispersion of the nuclear envelopes and/or a short (*e.g.* less than about 2.5 hours, less than about 2 hours, less than about 1.5 hours, less than about 1 hour, less than about 30 minutes, or less than about 15 minutes) time period between syngamy and the beginning of the first cytokinesis ($P_{syn}$). Therefore, in one embodiment, the time period between syngamy and the beginning of the first cytokinesis ($P_{syn}$) is measured. In a particular embodiment, embryos with a short time period between syngamy and the first cytokinesis ($P_{syn}$) are less likely to reach the blastocyst stage or implant into the uterus or be born live or are more likely to be aneuploid and therefore are deselected. In some embodiment, embryos with a shorter time period between syngamy and the first cytokinesis ($P_{syn}$) are less likely to reach the blastocyst stage or to develop into a good quality blastocyst, or implant into the uterus or be born live or are more likely to be aneuploid. These embryos show lower potential of development and may have lower potential to implant into the uterus, be born live and/or may be more likely to be aneuploid and therefore are deselected. In some embodiments, embryos are deselected as being less likely to reach the blastocyst stage or implant into the uterus or be born live and/or more likely to be aneuploid when $P_{syn}$ is immeasurable.

In some embodiments, in addition to measuring $P_{syn}$, or observing immeasurable syngamy or AS, one or more additional cellular parameters are measured including: the duration of P1 or first cytokinesis (i.e. the time period between the appearance of the 1st cleavage furrow to completion of the 1st cell division) and/or one or more P1 phenotypes (e.g. membrane ruffling, oolemma ruffling and/or formation of pseudo cleavage furrows prior to completing the first cytokinesis (P1)); and/or the duration of a cytokinesis event, e.g. the duration of cytokinesis 1, the time interval between cytokinesis 1 and cytokinesis 2; or the time interval between cytokinesis 2 and cytokinesis 3. In some embodiments, the one or more cellular parameters is: the duration of a mitotic event, e.g. the time interval between mitosis 1 and mitosis 2; and the time interval between mitosis 2 and mitosis 3. In certain embodiments, the duration of cell cycle 1 is also utilized as a cellular parameter. In certain embodiment, the time between fertilization and the 5 cell stage is also utilized as a cellular parameter. In certain embodiments, the presence or absence of abnormal cleavage (AC) and/or chaotic cleavage is utilized as a cellular parameter. In some embodiments, the cell parameter measurement is employed by comparing it to a comparable cell parameter measurement from a reference embryo, and using the result of this comparison to provide a determination of the likelihood of the embryo to reach the blastocyst stage and/or become a good quality blastocyst and/or implant into the uterus and/or be born live and/or be euploid. In some embodiments, the embryo is a human embryo.

In some aspects of the invention, methods are provided for deselecting one or more human embryos with poor developmental potential and/or are not likely to reach the blastocyst stage and/or not likely to become good quality blastocyst and/or less likely to implant into the uterus and/or are more likely to be aneuploid when the embryo displays abnormal cleavage (AC). In one embodiment, the embryo is deselected when it displays AC1 and/or AC2. In one embodiment, the embryo is deselected when it displays AC2.

In some aspects of the invention, methods are provided for selecting one or more human embryos that is likely to reach the blastocyst stage or become a good quality blastocyst or successfully implant into the uterus or be born live or be euploid by culturing one or more human embryos under conditions sufficient for embryo development. In certain embodiments, the embryos are frozen prior to culturing. In other embodiments, the embryos are not frozen prior to culturing. In certain embodiments, the one or more human embryos are produced by fertilization of oocytes *in vitro.* In further embodiments, the oocytes that are fertilized *in vitro* are also matured in vitro and may be supplemented with growth factors. In certain embodiments, the one or more human embryos that is cultured under conditions sufficient for embryo development is further imaged by time lapse imaging for a duration sufficient to include at least one cytokinesis event or cell cycle. In a particular embodiment, the time lapse imaging acquires images that are digitally stored. In one embodiment, the time lapse imaging employs darkfield illumination. In another embodiment, the time lapse imaging employs brightfield illumination. In still a further embodiment, the time lapse imaging employs a combination of darkfield and brightfield illumination. In one embodiment, the time-lapse imaging employs single plane acquisition. In another embodiment, the time-lapse imaging employs multi-plane acquisition. In one embodiment, one or more cellular parameters is measured by time lapse microscopy. In one embodiment, the one or more cellular parameters is the duration of the first cytokinesis, the time interval between the first and second mitosis, the time interval between the second and third mitosis, the time interval between cytokinesis 1 and cytokinesis 2, the time interval between cytokinesis 2 and cytokinesis 3, the duration of the first cell cycle and the time between fertilization and the 5 cell stage. In still a further embodiment, an embryo is selected when the duration of the first cytokinesis is about 0 to about 33 minutes or the time interval between mitosis 1 and mitosis 2 is about 7.8 to about 14.3 hours, or the time interval between mitosis 2 and mitosis 3 is about 0 to about 5.8 hours or the time interval between the first cytokinesis and the second cytokinesis is about 7.8 to about 14.3 hours, or the time interval between cytokinesis 2 and cytokinesis 3 is about 0 to about 5.8 hours, or the duration of the first cell cycle is about 24 hours or the time between fertilization and the 5 cell stage is about 47 to about 57 hours. In still a further embodiment, a human embryo selected to be more likely to reach the blastocyst stage or implant into the uterus or be born live or be euploid (i.e. more likely to be aneuploid) is deselected when the embryo displays A1$^{cyt}$, AS, US, AC and/or chaotic cleavage.

In some aspects of the invention, methods are provided to select the best embryos that are most likely to be euploid, reach blastocyst stage, and/or develop into good quality blastocysts, and/or have higher potential of development and/or implant into the uterus, and/or be born live, by culturing human embryos *in vitro,* time-lapse imaging the embryos to measure cellular parameters, employing the cellular parameters to determine the likelihood of the embryo reaching blastocyst, becoming a good quality blastocyst, implanting into the uterus and/or being born live and further by deselecting embryos that fall within certain other cellular parameters that make it less likely that the one or more human embryo will reach the blastocyst stage, implant into the uterus and/or be born live and/or are more likely to be aneuploid.

BRIEF DESCRIPTION OF THE DRAWINGS

The invention is best understood from the following detailed description when read in conjunction with the accompanying drawings. It is emphasized that, according to common practice, the various features of the drawings are not to-scale. On the contrary, the dimensions of the various features are arbitrarily expanded or reduced for clarity. Included in the drawings are the following figures.

Figure 1 describes and illustrates the definition of each of the four atypical phenotypes and describes their prevalence..

Figure 2 depicts individual still images from key time-points occurring during the dynamic atypical phenotype event, including an example of an embryo exhibiting more than one phenotype.

DETAILED DESCRIPTION OF THE INVENTION

Before the present methods and compositions are described, it is to be understood that this invention is not limited to any particular method or composition described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, some potential and preferred methods and materials are now described. All publications mentioned herein are incorporated herein by reference to disclose and describe the methods and/or materials in connection with which the publications are cited. It is understood that the present disclosure supersedes any disclosure of an incorporated publication to the extent there is a contradiction.

It must be noted that as used herein and in the appended claims, the singular forms "a", "an", and "the" include plural referents unless the context clearly dictates otherwise. Thus, for example, reference to "a cell" includes a plurality of such cells and reference to "the peptide" includes reference to one or more peptides and equivalents thereof, e.g. polypeptides, known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

Methods, compositions and kits for determining the likelihood of reaching the blastocyst stage and/or implant into the uterus. These methods, compositions and kits find use in identifying embryos in vitro that are most useful in treating infertility in humans. These and other objects, advantages, and features of the invention will become apparent to those persons skilled in the art upon reading the details of the subject methods and compositions as more fully described below.

The terms "developmental potential" and "developmental competence' are used herein to refer to the ability or capacity of a healthy embryo or to grow or develop. The terms may refer to the ability or capacity of a healthy embryo to reach the blastocyst stage, or develop into a good quality blastocyst or implant into the uterus, or be born live.

The term "specificity" when used herein with respect to prediction and/or evaluation methods is used to refer to the ability to predict or evaluate an embryo for determining the likelihood that the embryo will not develop into a blastocyst by assessing, determining, identifying or selecting embryos that are not likely to reach the blastocyst stage and/or implant into the uterus. High specificity as used herein refers to where at least about 70%, 72%, 75%, 77%, 80%, 82%, 85%, 88%, 90%, 92%, 95% or more, or 100% of the human embryos not selected are not likely to reach the blastocyst stage and/or implant into the uterus. In some embodiments, embryos that are not likely to reach the blastocyst stage and/or implant into the uterus stage are deselected.

The term "embryo" is used herein to refer both to the zygote that is formed when two haploid gametic cells, e.g. an unfertilized secondary oocyte and a sperm cell, unite to form a diploid totipotent cell, e.g. a fertilized ovum, and to the embryo that results from the immediately subsequent cell divisions, i.e. embryonic cleavage, up through the morula, i.e. 16-cell stage and the blastocyst stage (with differentiated trophectoderm and inner cell mass).

The term "blastocyst" is used herein to describe all embryos that reach cavitation (i.e., the formation of cavities).

The terms "born live" or "live birth" are used herein to include but are not limited to healthy and/or chromosomally normal (normal number of chromosomes, normal chromosome structure, normal chromosome orientation, etc.) births.

The term "arrested" is used herein to refer to any embryo that does not meet the definition of blastocyst.

The term "oocyte" is used herein to refer to an unfertilized female germ cell, or gamete. Oocytes of the subject application may be primary oocytes, in which case they are positioned to go through or are going through meiosis I, or secondary oocytes, in which case they are positioned to go through or are going through meiosis II.

By "meiosis" it is meant the cell cycle events that result in the production of gametes. In the first meiotic cell cycle, or meiosis I, a cell's chromosomes are duplicated and partitioned into two daughter cells. These daughter cells then divide in a second meiotic cell cycle, or meiosis II, that is not accompanied by DNA synthesis, resulting in gametes with a haploid number of chromosomes.

By a "mitotic cell cycle", it is meant the events in a cell that result in the duplication of a cell's chromosomes and the division of those chromosomes and a cell's cytoplasmic matter into two daughter cells. The mitotic cell cycle is divided into two phases: interphase and mitosis. In interphase, the cell grows and replicates its DNA. In mitosis, the cell initiates and completes cell division, first partitioning its nuclear material, and then dividing its cytoplasmic material and its partitioned nuclear material (cytokinesis) into two separate cells.

By a "first mitotic cell cycle" or "cell cycle 1" it is meant the time interval from fertilization to the completion of the first cytokinesis event or first mitosis, i.e. the division of the fertilized oocyte into two daughter cells. In instances in which oocytes are fertilized in vitro, the time interval between the injection of human chorionic gonadotropin (HCG) (usually administered prior to oocyte retrieval) to the completion of the first cytokinesis event may be used as a surrogate time interval.

"P1" or "P1 duration" is used herein to refer to the time interval between the appearance of the first cleavage furrow to completion of the 1st cell division or first cytokinesis event.

"1st cytokinesis phenotype" or "P1 phenotype" is used herein to refer to the cellular, biochemical and/or morphological characteristics of an embryo prior to completing P1 (i.e. the cellular, physical, biochemical and/or morphological characteristics of an embryo prior to completing the 1st cell division or first cytokinesis event).

"Abnormal P1 phenotype" or "A1$^{cyt}$" is used herein to refer to uncharacteristic cellular, biochemical and/or morphological events of an embryo prior to completing P1 (i.e. prior to completing the 1st cell division or first cytokinesis event) when compared to a reference or control embryo having a high likelihood of reaching blastocyst, becoming a good quality blastocyst and/or implanting into the uterus. "Abnormal phenotype" or "abnormal P1 phenotype" or "A1$^{cyt}$" as used herein includes oolemma ruffling, membrane ruffling, and/or formation of one or more pseudo cleavage furrows before the initiation and/or completion of P1 (the time interval between the appearance of the first cleavage furrow to completion of the 1st cell division or first cytokinesis event). By "oolema ruffling" is meant a phenomenon when the oolema membrane becomes irregular and unsmooth over its entire surface.

By a "second mitotic cell cycle" or "cell cycle 2" or "P2" it is meant the second cell cycle event observed in an embryo, the time interval between the production of daughter cells from a fertilized oocyte by mitosis and the production of a first set of granddaughter cells from one of those daughter cells (the "leading daughter cell", or daughter cell A) by mitosis. P2 also encompasses the duration of time that the embryo is a 2 cell embryo, that is the duration of the 2 cell stage. Cell cycle 2 may be measured using several morphological events including the end of cytokinesis 1 and the beginning of cytokinesis 2, or the end of cytokinesis 1 and the end of cytokinesis 2 or the beginning of cytokinesis 1 and the beginning of cytokinesis 2 or the beginning of cytokines 1 and the end of cytokinesis 2 or the end of mitosis 1 and the beginning of mitosis 2 or the end of mitosis 1 and the end of mitosis 2 or the beginning of mitosis 1 and the beginning of mitosis 1 or the beginning of mitosis 1 and the end of mitosis 2. Upon completion of cell cycle 2, the embryo consists of 3 cells. In other words, cell cycle 2 can be visually identified as the time between the embryo containing 2-cells and the embryo containing 3-cells.

By a "third mitotic cell cycle" or "cell cycle 3" or "P3" it is meant the third cell cycle event observed in an embryo, typically the time interval from the production of a first set of granddaughter cells from a fertilized oocyte by mitosis and the production of a second set of granddaughter cells from the second daughter cell (the "lagging daughter cell" or daughter cell B) by mitosis. Cell cycle 3 may be measured using several morphological events including the end of cytokinesis 2 and the beginning of cytokinesis 3, or the end of cytokinesis 2 and the end of cytokinesis 3 or the beginning of cytokinesis 2 and the beginning of cytokinesis 3 or the beginning of cytokinesis 2 and the end of cytokinesis 3 or the end of mitosis 3 and the beginning of mitosis3 or the end of mitosis 2 and the end of mitosis3 or the beginning of mitosis 2 and the beginning of mitosis 3 or the beginning of mitosis 2 and the end of mitosis 3. In other words, cell cycle 3 can be visually identified as the time between the embryo containing 3-cells and the embryo containing 4-cells.

By "first cleavage event" or "first cleavage", it is meant the first division, i.e. the division of the oocyte into two daughter cells, i.e. cell cycle 1. Upon completion of the first cleavage event, the embryo consists of 2 cells.

By "second cleavage event" or "second cleavage", it is meant the second set of divisions, i.e. the division of leading daughter cell into two granddaughter cells and the division of the lagging daughter cell into two granddaughter cells. In other words, the second cleavage event consists of both cell cycle 2 and cell cycle 3. Upon completion of second cleavage, the embryo consists of 4 cells.

By "third cleavage event", it is meant the third set of divisions, i.e. the divisions of all of the granddaughter cells. Upon completion of the third cleavage event, the embryo typically consists of 8 cells.

By "cytokinesis" or "cell division" it is meant that phase of mitosis in which a cell undergoes cell division. In other words, it is the stage of mitosis in which a cell's partitioned nuclear material and its cytoplasmic material are divided to produce two daughter cells. The period of cytokinesis is identifiable as the period, or window, of time between when a constriction of the cell membrane (a "cleavage furrow") is first observed and the resolution of that constriction event, i.e. the generation of two daughter cells. The initiation of the cleavage furrow may be visually identified as the point in which the curvature of the cell membrane changes from convex (rounded outward) to concave (curved inward with a dent or indentation). This is illustrated for example in Fig.4 of US Patent No. 7,963,906 top panel by white arrows pointing at 2 cleavage furrows. The onset of cell elongation may also be used to mark the onset of cytokinesis, in which case the period of cytokinesis is defined as the period of time between the onset of cell elongation and the resolution of the cell division.

By "first cytokinesis" or "cytokinesis 1" it is meant the first cell division event after fertilization, i.e. the division of a fertilized oocyte to produce daughter cells. First cytokinesis usually occurs about one day after fertilization.

By "second cytokinesis" or "cytokinesis 2", it is meant the second cell division event observed in an embryo, i.e. the division of a daughter cell of the fertilized oocyte (the "leading daughter cell", or daughter A) into a first set of granddaughters.

By "third cytokinesis" or "cytokinesis 3", it is meant the third cell division event observed in an embryo, i.e. the division of the other daughter of the fertilized oocyte (the "lagging daughter cell", or daughter B) into a second set of granddaughters.

The term "fiduciary marker" or "fiducial marker," is an object used in the field of view of an imaging system which appears in the image produced, for use as a point of reference or a measure. It may be either something placed into or on the imaging subject, or a mark or set of marks in the reticle of an optical instrument.

The term "micro-well" refers to a container that is sized on a cellular scale, preferably to provide for accommodating eukaryotic cells or a single oocyte or embryo.

The term "selecting" or "selection" refers to any method known in the art for moving one or more embryos, blastocysts or other cell or cells as described herein from one location to another location. This can include but is not limited to moving one or more embryos, blastocysts or other cell or cells within a well, dish or other compartment or device so as to separate the selected one or more embryos, blastocysts or other cell or cells of the invention from the non- or deselected one or more embryos of the invention (such as for example moving from one area of a well, dish, compartment or device to another area of a well, dish, compartment or device). This can also include moving one or more embryos, blastocysts or other cell or cells from one well, dish, compartment or device to another well, dish, compartment or device. Any means known in the art for separating or distinguishing the selected one or more embryos, blastocysts or other cell or cells from the non- or deselected one or more embryos, blastocysts or other cell or cells can be employed with the methods of the present invention. In one embodiment, selected embryos are selected for transfer to a recipient for gestation. In another embodiment, selected embryos are selected for freezing for potential future transfer. In another embodiment, embryos are selected for continued culture. In another embodiment, embryos are selected for further evaluation by other methods such as preimplantation genetic testing, genomics, proteonomics, and/or secretomics.

The term "deselected" or "deselection" as used herein refers to embryos with poor developmental potential which not chosen for transfer or are chosen for non-transfer. In some embodiments, deselected embryos are not transferred into the uterus.

The term "euploid" is used herein to refer to a cell that contains an integral multiple of the haploid, or monoploid, number. For example, a human autosomal cell having 46 chromosomes is euploid, and a human gamete having 23 chromosomes is euploid. By "euploid embryo" it is meant that the cells of the embryo are euploid. The terms "euploid" and "chromosomally normal" are used herein interchangeably.

The term "aneuploid" is used herein to refer to a cell that contains an abnormal number of chromosomes. For example, a cell having an additional chromosome and a cell missing a chromosome are both aneuploid. By "aneuploid embryo" it is meant that one or more cells of an embryo are aneuploid. The terms "aneuploid" and "chromosomally abnormal" are used herein interchangeably.

After fertilization both gametes contribute one set of chromosomes (haploid content), each contained in a structured referred to herein as a "pronucleus." ("PN") After normal fertilization, each embryo shows two pronuclei (PNs), one representing the paternal genetic material and one representing the maternal genetic material. "Syngamy" as used herein refers to the breakdown or disappearance of the pronuclei (PNs) when the two sets of chromosomes unite, occurring within a couple hours before the first cytokinesis.

The time parameter "$P_{syn}$" or "S" or "$P_{M1}$", as used interchangeably herein, refers to a parameter defined by the time from syngamy to the beginning of the first cytokinesis (i.e., the appearance of the first cytokinetic cleavage furrow). Sometimes it is not possible to visualize PN or to measure syngamy, such embryos are said to have "immeasurable syngamy" or "unmeasurable syngamy" or "US" (all terms are used interchangeably). Additionally, it is possible that an embryo will show atypical syngamy patterns or timing. Such embryos are said to have "atypical syngamy" or "abnormal syngamy" or "AS" (all three terms are used interchangeably). AS embryos show disordered PN movement within the cytoplasm without prompt dispersion of nuclear envelopes and typically have an average shorter $P_{syn}$, when compared to normal syngamy or "NS" embryos. This may be visualized by time lapse microscopy when the PN move unsteadily within the cytoplasm either together or separately before their disappearance. AS embryos often also show active oolema movement before the dispersion of the nuclear envelopes. NS embryos on the other hand, show timely disappearance of PNs with a smooth dispersion of the nuclear envelopes with minimal or no pronuclear movement within the cytoplasm and minimal or no oolema movement prior to the dispersion of nuclear envelopes.

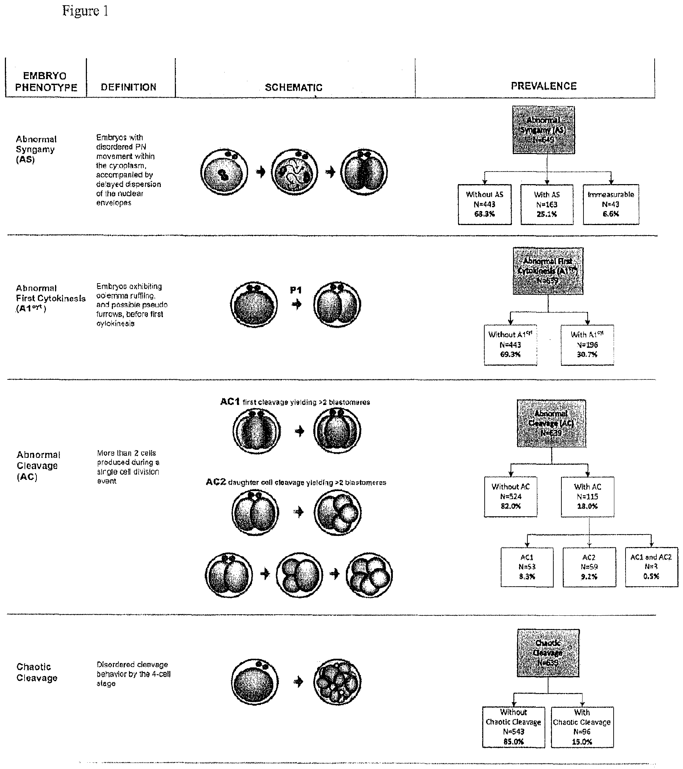

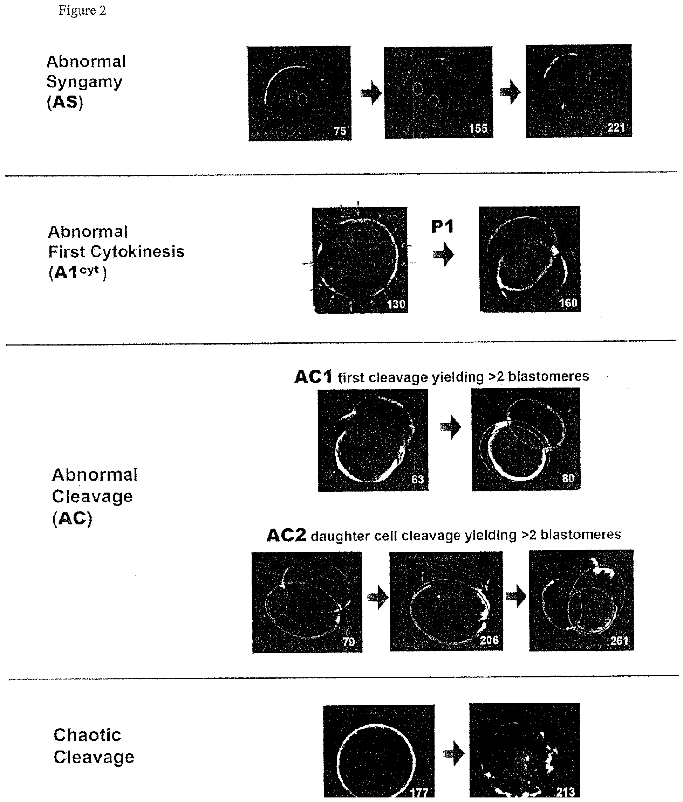

The parameter "AC" as used herein refers to abnormal cleavages wherein more than two cells originate from a single cleavage. For example, when one blastomere gives rise to more than two daughter cells, the embryo is referred to as an AC embryo or the embryo is said to display AC. By "AC1" is meant that the AC phenotype happens at the first cleavage. In an AC1 embryo a single cell embryo divides once and gives rise to a three or more cell embryo (e.g. 1→3 cells) (Figure 1 and Figure 2). By "AC2" is meant that the AC phenotype happens at the second cleavage. In an AC2 embryo, a single blastomere divides to give rise to three or more cells (e.g. 1→4 cells) (Figure 1 and Figure 2) during the second cleavage. By "AC3" is meant that the AC phenotype happens at the third cleavage. In an AC3 embryo, a single blastomere of a three cell embryo divides to give rise to three or more cells. AC can happen at any cleavage, and/or during more than one cleavage event. For example, an AC1 embryo (e.g. 1→3 cells) can also display AC2 (e.g. 3→5 cells).

The atypical phenotype herein referred to as "chaotic cleavage" means a cleavage phenotype distinguished by the appearance of disordered cleavage or cell division behavior by the 4-cell stage. Chaotic cleavage may be visualized using time-lapse microscopy when the first cell divisions are erratic and frequently result in uneven-sized blastomeres and/or fragments.

Single-embryo transfer is the preferred practice in vitro fertilization treatment, as it reduces the risk for adverse outcomes associated with multiple gestation pregnancy. However, to improve pregnancy rates for SET, embryologists need reliable assessment tools to aid in embryo selection so embryos with the highest developmental potential can be selected, while those with lower developmental potentials arc not selected or are deselected. Current embryo selection methods are based on morphological evaluations, which use static observation of cell number and shapes and are highly subjective. Therefore, morphology assessment offers limited predictive value of embryo viability.

Abnormal cleavage or AC arises when more than two cells, for example three cells, or four cells or five or more cells, originates from a single cleavage event. For example, AC1 arises when a single cell embryo divides once and gives rise to three daughter cells, thereby forming a three cell embryo (Figure 1 and Figure 2). AC2 arises when one blastomere divides once during the second cleavage to give rise to more than two daughter cells (Figure 1 and Figure 2). While it is common that the AC phenotype is characterized by the division of one cell into three cells, AC phenotypes also encompass division of once cell into more than three cells, for example 4 cells, or 5 or more cells. Additionally, it is important to note that AC2 may be present in an AC1 embryo. In such an instance, AC2 is may be characterized by the cleavage of a three cell embryo into a five or more cell embryo during the second cleavage and by one cleavage event. The numerical designation following the "AC" refers not to the number of cells in the embryo displaying AC, but rather to the cleavage event in which the AC occurs. That is, AC1 embryos display AC in the first cleavage event while AC2 embryos display AC in the second cleavage event. Detection of AC patterns during pre-implantation embryo development are useful in deselecting embryos with low implantation potential and low blastocyst formation rate. Conversely, detection of the lack of AC patterns during pre-implantation embryo development are similarly useful in selecting embryos that are more likely to reach blastocyst stage and/or become a good quality blastocyst and/or successfully implant into the uterus and/or are euploid.

As described herein, AC1 and AC2 embryos show low blastocyst formation rate and lower implantation potential when compared to embryos without this phenotype. However, surprisingly, a very high percentage of AC embryos are considered to have good morphology on day 3 and therefore may be selected for transfer or freezing for later transfer if the AC phenotype is not identified by the embryologist during pre-implantation embryo culture. Therefore, in accordance with the current invention, methods are provided to allow embryologists who have selected an embryo as being of high quality on day 3, to deselect embryos with AC and further improve implantation rates. Furthermore, methods are provided for selecting an embryo for implantation into the uterus when the embryo does not display AC.

Direct cleavage phenotypes have been described in the art, for example by Rubio, et al. (2012) "Limited Implantation Success of Direct-Cleaved Human Zygotes: A Time-Lapse Study," Fertil. Steril., 98:1458-63, and Campbell, et al. (2013) "Modeling a Risk of Classification of Anneuploidy in Human Embryos Using Non-Invasive Morphokinetics," Reprod. Bioemed. Online. However, Rubio only examines direct cleavage as a function of the duration of time spent as a 2 cell embryo and neither Rubio nor Campbell teaches or suggests using AC as a deselection parameter of embryos otherwise selected to have good developmental potential as described herein.

The focus of prior patents and applications including US Patent No.: 7,963,906; 8,323,177; 8,337,387 and PCT Appl. No. WO 2012/163363 each center primarily around selection criteria for human embryos in in vitro fertilization. While these patents/applications each discuss determining whether embryos are good or poor, the timing parameters described therein are typically used in the clinic in large part to select embryos with good developmental potential. In contrast, the methods of the current invention center around the novel parameters, including, for example, prolonged duration of P1 or first cytokinesis (i.e. the time period between the appearance of the 1st cleavage furrow to completion of the 1st cell division) P1 $\geq$0.5hr and/or one or more abnormal P1 phenotypes (A1$^{cyt}$) (including, e.g., membrane ruffling, oolemma ruffling and/or formation of one or more pseudo cleavage furrows prior to completing the first cytokinesis (P1)) and/or abnormal cleavage (AC), that may be used to deselect human embryos and target them for non-transfer in in vitro fertilization treatment. These parameters may be used alone or in combination with each other or selection parameters including syngamy parameters, and chaotic cleavage as well as the selection parameters described in US Patent No.: 7,963,906; 8,323,177; 8,337,387 and PCT Appl. No. WO 2012/163363. For example, once an embryo is determined to have good developmental potential by the methods of US Patent No.: 7,963,906; 8,323,177; 8,337,387 and PCT Appl. No. WO 2012/163363, that embryo may be further analyzed for the novel P1 parameters, AC, AS, US and/or chaotic cleavage parameters described herein to further increase the sensitivity and specificity of the claimed methods.

The deselection criteria or atypical phenotypes of the current invention include, A1$^{cyt}$ or prolonged duration of P1 or first cytokinesis (i.e. the time period between the appearance of the 1st cleavage furrow to completion of the 1st cell division) where P1 $\geq$0.5hr and/or one or more abnormal P1 phenotypes (including, e.g., membrane ruffling, oolemma ruffling and/or formation of pseudo cleavage furrows prior to completing the first cytokinesis (P1)); AS or abnormal syngamy, an abnormal embryo phenotype involving pronuclear behavior that can be measuring during the physiological process called syngamy, embryos exhibiting this type of phenotype are considered to have abnormal syngamy; immeasurable syngamy, an abnormal embryo phenotype identified by non-visualization of the pronuclei; AC or abnormal cleavage which occurs when one blastomere divides to give rise to more than two daughter cells; and chaotic cleavage, or disordered cleavage behavior by the 4-cell stage, often resulting in uneven blastomeres and/or fragments. See Figure 1 and Figure 2. Any one of these parameters, AC (e.g. AC1 and/or AC2), A1$^{cyt}$, AS, US and/or chaotic cleavage may be used alone or in combination with each other or other cellular parameters including the parameters included in Table 1.

Table 1: List of Parameters

| Parameter | Description/Reference describing Parameter |
|---|---|
| P1 | Duration of 1st cytokinesis |

(continued)

| Parameter | Description/Reference describing Parameter |
|---|---|
| P1 Phenotypes (A1$^{cyt}$) | Membrane ruffling, oolemma ruffling and/or formation of one or more pseudo cleavage furrows prior to completing the first cytokinesis (P1) |
| P2 | Interval between 1$^{st}$ and 2$^{nd}$ cytokinesis (time from 2-cell embryo to 3-cell embryo) (end of 1$^{st}$ cytokinesis to end of 2$^{nd}$ cytokinesis) (duration as 2 cell embryo) (t3-t2) |
| P3 | Interval between 2$^{nd}$ and 3$^{rd}$ cytokinesis (time from 3-cell embryo to 4-cell embryo) (end of 2$^{nd}$ cytokinesis to end of 3$^{rd}$ cytokinesis) (duration as 3 cell embryo) (t4-t3) (synchrony between 3 and 4 cells) |
| P$_{syn}$ or S or P$_{M1}$ | Time from syngamy to 1$^{st}$ cytokinesis (appearance of the first cytokinetic cleavage furrow) |
| 2ce-3C | End of 1$^{st}$ cleavage to beginning of second cleavage |
| 3C-4C | Beginning of 2$^{nd}$ Cleavage to end of 3$^{rd}$ Cleavage |
| t5 | Time from ICSI (insemination) to 5 cell embryo |
| 2Cb | Time from insemination to beginning of 1$^{st}$ cleavage |
| 2Ce | Time from insemination until end of 1$^{st}$ cleavage |
| 3C | Time from insemination to beginning of 2$^{nd}$ cleavage |
| 4C | Time from insemination to end of 3$^{rd}$ cleavage |
| 5C | Time from insemination to beginning of 4$^{th}$ cleavage |
| BL | Formation of blastocoel |
| tM | Time from fertilization to morula |
| S3 | Time from 5 cell embryo to 8 cell embryo |
| t2 | Time from insemination to 2 cell embryo |
| t3 | Time from insemination to 3 cell embryo |
| t4 | Time from insemination to 4 cell embryo |
| cc3 | T5-t3: Third cell cycle, duration of period as 3 and 4 cell embryo |
| t5-t2 | Time to 5 cell embryo minus time to 2 cell embryo |
| cc3/cc2 | Ratio of duration of cell cycle 3 to duration of cell cycle 2 |
| Time till first cleavage | Duration of 1$^{st}$ cell cycle |
| 2PB Extrusion | Time from insemination until the second polar body is extruded |
| PN fading | Time from insemination until pronuclei disappear, OR time between the appearance of pronuclei appearing and pronuclei disappearing |
| tSB | Time from insemination to the start of blastulation |
| tSC | Time from insemination to the start of compaction |
| PN appearance | Time from insemination until pronuclei appear |
| t6 | Time from insemination to 6 cell embryo |
| t7 | Time from insemination to 7 cell embryo |
| t8 | Time from insemination to 8 cell embryo |
| cc2b | t4-t2; Second cell cycle for both blastomeres, duration of period as 2 and 3 cell blastomere embryo |
| cc2_3 | t5-t2; Second and third cell cycle, duration of period as 2, 3, and 4 blastomere embryo |
| cc4 | t9-t5; fourth cell cycle; duration of period as 5, 6, 7 and 8 blastomere embryo. |

(continued)

| Parameter | Description/Reference describing Parameter |
|---|---|
| s3a | t6-t5; Duration of the individual cell divisions involved in the development from 4 blastomere embryo to 8 blastomere embryo |
| s3b | t7-t6; Duration of the individual cell divisions involved in the development from 4 blastomere embryo to 8 blastomere embryo |
| s3c | t8-t7; Duration of the individual cell divisions involved in the development from 4 blastomere embryo to 8 blastomere embryo |
| cc2/cc3 | WO 2012/163363 |
| cc2/cc2_3 | WO 2012/163363 |
| cc3/t5 | WO 2012/163363 |
| s2/cc2 | WO 2012/163363 |
| s3/cc3 | WO 2012/163363 |
| AC1 | Cleavage directly from 1 cell embryo to 3 or more cell embryo |
| AC2 | Cleavage of a daughter cell into more than 2 blastomeres |
| AS | Abnormal syngamy Disordered PN movement within the cytoplasm without prompt dispersion of nuclear envelopes, short time period between syngamy and the beginning of the first cytokinesis ($P_{syn}$), and/or active oolema movement before the dispersion of the nuclear envelopes. Measurable by evaluating the movement of pronuclei and/or pronuclei activity throughout the cytoplasm. |
| MN2 | Multinucleation observed at 2 blastomere stage |
| MN4 | Multinucleation observed at 4 blastomere stage |
| EV2 | Evenness of the blastomeres in the 2 blastomere embryo |
| Mul | Multinucleation |
| Uneven or UBS | Uneven sizes of blastomeres at 2-4 cells |
| Frg | Fragmentation |
| Nec | Blastomere necrosis |
| Vac | Vacuolization |

Previous reports have investigated the time from insemination to pronuclei disappearance, also known as pronuclei breakdown (PNB) or pronuclear fading (PNF), (Basile, et al. (2013) "Type of Culture Media Does Not Affect Embryo Kinetics: A Time-Lapse Analysis of Sibling Oocytes," Human Reprod., 28(3):634-41; Azzarello, et al. (2012) "The Impact of Procuclei Morphology and Dynamicity on Live Birth Outcome After Time-Lapse Culture," Human Reprod., 27(9):2649-57; Lemmen, et al. (2008) "Kinetic Markers of Human Embryo Quality Using Time-Lapse Recordings of IVF/ICSI-Fertilize Oocytes," Reprod. Biomed. Online, 17(3):385-91). Some methods of the current invention, in contrast, are related to the timing from PN disappearance to the first cytokinesis, $P_{syn}$. Unlike the previously described parameters, $P_{syn}$ is a more reliable measurement since it does not rely on the time of insemination. Time of insemination can be imprecise, especially for eggs inseminated under classic in vitro fertilization techniques.

The methods of the current invention, therefore, provide for novel selection or deselection cellular parameters for human embryos that can be measured by time lapse microscopy.

In methods of the invention, one or more embryos is assessed for its likelihood to reach the blastocyst stage and/or become a good quality blastocyst and/or implant into the uterus and/or be euploid by measuring one or more cellular parameters of the embryo(s) and employing these measurements to determine the likelihood that the embryo(s) will reach the blastocyst stage or implant into the uterus. Such parameters, are described herein (NS, AS, US, AC A1$^{cyt}$, and chaotic cleavage) and have been described, for example, in US Patent Nos. 7,963,906; 8,323,177, and 8,337,387 and PCT Appl. No.: WO 2012/163363, the disclosure of each of which is incorporated herein by reference in their entirety. The information thus derived may be used to guide clinical decisions, e.g. whether or not to transfer an in vitro fertilized embryo, whether or not to transplant a cultured cell or cells, whether or not to freeze an embryo for later implantation, whether or not to continue to culture the embryo, or whether or not to evaluate the embryo by other methods such as preimplantation genetic testing, genomics, proteonomics, and/or secretomics.

Examples of embryos that may be assessed by the methods of the invention include 1-cell embryos (also referred to as zygotes), 2-cell embryos, 3-cell embryos, 4-cell embryos, 5-cell embryos, 6-cell embryos, 8-cell embryos, etc. typically up to and including 16-cell embryos, morulas, and blastocysts, any of which may be derived by any convenient manner, e.g. from an oocyte that has matured in vivo or from an oocyte that has matured in vitro.

Embryos may be derived from any organism, e.g. any mammalian species, e.g. human, primate, equine, bovine, porcine, canine, feline, etc. Preferable, they are derived from a human. They may be previously frozen, e.g. embryos cryopreserved at the 1-cell stage and then thawed. Alternatively, they may be freshly prepared, e.g., embryos that are freshly prepared (not frozen prior to culturing) from oocytes by in vitro fertilization techniques (fresh or previously frozen oocytes); oocytes that are freshly harvested and/or freshly matured through in vitro maturation techniques (including, e.g., oocytes that are harvested from in vitro ovarian tissue). They may be cultured under any convenient conditions (including different types of culture media) known in the art to promote survival, growth, and/or development of the sample to be assessed, e.g. for embryos, under conditions such as those used in the art of in vitro fertilization; see, e.g., US Patent No. 6,610,543, US Patent No. 6,130,086, US Patent No. 5,837,543, the disclosures of which are incorporated herein by reference; for oocytes, under conditions such as those used in the art to promote oocyte maturation; see, e.g., US Patent No. 5,882,928 and US Patent No. 6,281,013, the disclosures of which are incorporated herein by reference; for stem cells under conditions such as those used in the art to promote maintenance, differentiation, and proliferation, see, e.g. US Patent No. 6,777,233, US Patent No. 7,037,892, US Patent No. 7,029,913, US Patent No. 5,843,780, and US Patent No. 6,200,806, US Application No. 2009/0047263; US Application No. 2009/0068742, the disclosures of which are incorporated herein by reference. Often, the embryos are cultured in a commercially available medium such as KnockOut DMEM, DMEM-F12, or Iscoves Modified Dulbecco's Medium that has been supplemented with serum or serum substitute, amino acids, growth factors and hormones tailored to the needs of the particular embryo being assessed.

In some embodiments, the embryos are assessed by measuring cell parameters by time-lapse imaging. The embryos may be cultured in standard culture dishes in vitro. Alternatively, the embryos may be cultured in custom culture dishes, e.g. custom culture dishes with optical quality micro-wells as described herein. In such custom culture dishes, each micro-well holds a single fertilized egg or embryo, and the bottom surface of each micro-well has an optical quality finish such that the entire group of embryos within a single dish can be imaged simultaneously by a single miniature microscope with sufficient resolution to follow the cell mitosis processes. The entire group of micro-wells shares the same media drop in the culture dish, and can also include an outer wall positioned around the micro-wells for stabilizing the media drop, as well as fiducial markers placed near the micro-wells. The media drops can have different volumes. The hydrophobicity of the surface can be adjusted with plasma etching or another treatment to prevent bubbles from forming in the micro-wells when filled with media. Regardless of whether a standard culture dish or a custom culture dish is utilized, during culture, one or more developing embryos may be cultured in the same culture medium, e.g. between 1 and 30 embryos may be cultured per dish.

Images are acquired over time, and are then analyzed to arrive at measurements of the one or more cellular parameters. Time-lapse imaging may be performed with any computer-controlled microscope that is equipped for digital image storage and analysis, for example, inverted microscopes equipped with heated stages and incubation chambers, or custom built miniature microscope arrays that fit inside a conventional incubator. The array of miniature microscopes enables the concurrent culture of multiple dishes of samples in the same incubator, and is scalable to accommodate multiple channels with no limitations on the minimum time interval between successive image capture. Using multiple microscopes eliminates the need to move the sample, which improves the system accuracy and overall system reliability. The individual microscopes in the incubator can be partially or fully isolated, providing each culture dish with its own controlled environment. This allows dishes to be transferred to and from the imaging stations without disturbing the environment of the other samples.

The imaging system for time-lapse imaging may employ brightfield illumination, darkfield illumination, phase contrast, Hoffman modulation contrast, differential interference contrast, polarized light, fluorescence or combinations thereof. In some embodiments, darkfield illumination may be used to provide enhanced image contrast for subsequent feature extraction and image analysis. In addition, red or near-infrared light sources may be used to reduce phototoxicity and improve the contrast ratio between cell membranes and the inner portion of the cells.

Images that are acquired may be stored either on a continuous basis, as in live video, or on an intermittent basis, as in time lapse photography, where a subject is repeatedly imaged in a still picture. Preferably, the time interval between images should be between 1 to 30 minutes, or between 1 to 20 minutes or between 1 to 15 minutes, or between 1 to 10 minutes or between 1 to 5 minute, minutes in order to capture significant morphological events as described below. In an alternative embodiment, the time interval between images could be varied depending on the amount of cell activity. For example, during active periods images could be taken as often as every few seconds or every minute, while during inactive periods images could be taken every 10 or 15 minutes or longer. Real-time image analysis on the captured images could be used to detect when and how to vary the time intervals. In our methods, the total amount of light received by the samples is estimated to be equivalent to approximately 52 seconds of continuous low-level light exposure for 5-days of imaging. The light intensity for a time-lapse imaging systems is significantly lower than the light intensity typically used on an assisted reproduction microscope due to the low-power of the LEDs (for example, using a 1W LED compared to a typical 100W Halogen bulb) and high sensitivity of the camera sensor. Thus, the total amount of light energy received by an embryo using the time-lapse imaging system is comparable to or less than the amount of energy received during routine handling at an IVF clinic. In addition, exposure time can be significantly shortened to reduce the total amount of light exposure to the embryo. For 2-days of imaging, with images captured every 5 minutes at 0.5 seconds of light exposure per image, the total amount of low-level light exposure is less than 21 seconds.

Following image acquisition, the images are extracted and analyzed for different cellular parameters, for example, zygote size, blastomeres size, thickness of the zona pellucida, smoothness or ruffling of the plasma membrane, smoothness or ruffling of the oolemma, formation of one or more pseudo cleavage furrows, degree of fragmentation, symmetry of daughter cells resulting from a cell division, time intervals between the first few mitoses, duration of cytokinesis and timing and quality of syngamy. The systems and methods, including classification, tracking and imaging modalities described in Patent Appln. Nos. PCT/US2011/053537; 61/785,170; 61/785,179; 61/785,199; 61/785,216; 61/770,998 and/or 61/771,000 may be used to observe and/or measure the cellular parameters of the current invention. The disclosures of each of these applications are herein specifically incorporated by reference in their entireties.

Cellular parameters that may be measured by time-lapse imaging are usually morphological events. For example, in assessing embryos, time-lapse imaging may be used to visualize the duration of P1 or first cytokinesis (i.e. the time period between the appearance of the 1st cleavage furrow to completion of the 1st cell division) and/or one or more P1 phenotypes (A1$^{cyt}$) including, e.g., membrane ruffling, oolemma ruffling and/or formation of one or more pseudo cleavage furrows prior to the initiation and/or completion of the first cytokinesis (P1). Additionally, time-lapse imaging may be used to visualize syngamy, particularly the timing of syngamy including the time between syngamy and the onset or resolution of cytokinesis 1, cytokinesis 2, cytokinesis 3, cytokinesis 4, or cytokinesis 5 or the time between syngamy and the onset or resolution of mitosis 1, mitosis 2, mitosis 3, mitosis 4, or mitosis 5. Additionally, time-lapse microscopy may be used to determine the number of cells arising from a single cell division, for example, to determine whether or not an embryo displays AC (e.g. AC1 and/or AC2). Additionally, time-lapse imaging may be used to measure the duration of a cytokinesis event, e.g. cytokinesis 1, cytokinesis 2, cytokinesis 3, cytokinesis 4, cytokinesis 5 or combinations and/or ratios of these events where the duration of a cytokinesis event is defined as the time interval between the first observation of a cleavage furrow (the initiation of cytokinesis) and the resolution of the cleavage furrow into two daughter cells (i.e. the production of two daughter cells). Another parameter of interest is the duration of a cell cycle event, e.g. cell cycle 1, cell cycle 2, cell cycle 3, cell cycle 4, cell cycle 5 or combinations and/or ratios of these events where the duration of a cell cycle event is defined as the time interval between the production of a cell (for cell cycle 1, the fertilization of an ovum; for later cell cycles, at the resolution of cytokinesis) and the production of two daughter cells from that cell. Cellular parameters of interest that can be measured by time-lapse imaging include time intervals that are defined by these cellular events, e.g. (a) the time interval between cytokinesis 1 and cytokinesis 2, definable as any one of the interval between initiation of cytokinesis 1 and the initiation of cytokinesis 2, the interval between the resolution of cytokinesis 1 and the resolution of cytokinesis 2, the interval between the initiation of cytokinesis 1 and the resolution of cytokinesis 2; or the interval between the resolution of cytokinesis 1 and the initiation of cytokinesis 2; or (b) the time interval between cytokinesis 2 and cytokinesis 3, definable as any one of the interval between the initiation of cytokinesis 2 and the initiation of cytokinesis 3, or the interval between resolution of the cytokinesis 2 and the resolution of cytokinesis 3, or the interval between initiation of cytokinesis 2 and the resolution of cytokinesis 3, or the interval between resolution of cytokinesis 2 and the initiation of cytokinesis 3; (c) the time interval between mitosis 1 and mitosis 2, definable as any one of the interval between initiation of mitosis 1 and the initiation of mitosis 2, the interval between the resolution of mitosis 1 and the resolution of mitosis 2, the interval between the initiation of mitosis 1 and the resolution of mitosis 2; or the interval between the resolution of mitosis 1 and the initiation of mitosis 2; or (b) the time interval between mitosis 2 and mitosis 3, definable as any one of the interval between the initiation of mitosis 2 and the initiation of mitosis 3, or the interval between resolution of the mitosis 2 and the resolution of mitosis 3, or the interval between initiation of mitosis 2 and the resolution of mitosis 3, or the interval between resolution of mitosis 2 and the initiation of mitosis 3. Other parameters that can be measured by time-lapse imaging include the presence or absence of atypical phenotypes. Such atypical phenotypes include, AC (i.e. AC1, AC2, AC3, AC4, etc), A1$^{cyt}$, AS, immeasurable syngamy and chaotic cleavage.

For the purposes of in vitro fertilization, it is considered advantageous that the embryo be transferred to the uterus early in development, e.g. by day 2, i.e. up through the 8-cell stage, to reduce embryo loss due to disadvantages of culture conditions relative to the in vitro environment, and to reduce potential adverse outcomes associated with epigenetic errors that may occur during culturing (Katari et al. (2009) Hum Mol Genet. 18(20):3769-78; Sepúlveda et al. (2009) Fertil Steril. 91(5):1765-70). Accordingly, it is preferable that the measurement of cellular parameters take place within 2 days of fertilization, although longer periods of analysis, e.g. about 36 hours, about 54 hours, about 60 hours, about 72 hours, about 84 hours, about 96 hours, or more, are also contemplated by the present methods.

Parameters can be measured manually, or they may be measured automatically, e.g. by image analysis software. When image analysis software is employed, image analysis algorithms may be used that employ a probabilistic model estimation technique based on sequential Monte Carlo method, e.g. generating distributions of hypothesized embryo models, simulating images based on a simple optical model, and comparing these simulations to the observed image data. When such probabilistic model estimations are employed, cells may be modeled as any appropriate shape, e.g. as collections of ellipses in 2D space, collections of ellipsoids in 3D space, and the like. To deal with occlusions and depth ambiguities, the method can enforce geometrical constraints that correspond to expected physical behavior. To improve robustness, images can be captured at one or more focal planes.

Once cell parameter measurements have been obtained, the measurements are employed to determine the likelihood that the embryo will develop into a blastocyst and/or become a good quality blastocyst and/or implant into the uterus and/or be euploid.

In some embodiments, the cell parameter measurement is used directly to determine the likelihood that an embryo will reach the blastocyst stage or will become a good quality embryo or will be euploid. In some embodiments, the cell parameter measurement is used directly to determine the likelihood that an embryo will successfully implant into the uterus and/or be euploid. In other words, the absolute value of the measurement itself is sufficient to determine the likelihood that an embryo will reach the blastocyst stage and/or implant into the uterus and/or be euploid. Examples of this in embodiments using time-lapse imaging to measure cellular parameters include, without limitation, the following, which in combination are indicative of the likelihood that an embryo will reach the blastocyst stage and/or implant into the uterus and/or be euploid: (a) a duration of cytokinesis that is about 0 to about 33 hours; (b) a time interval between the resolution of cytokinesis 1 and the onset of cytokinesis 2 that is about 8-15 hours, e.g. about 9-14 hours, about 9-13 hours, about 9-12 hours, or about 9-11.5 hours, or about 9.33-11.45 hours; and (c) a time interval, i.e. synchronicity, between the initiation of cytokinesis 2 and the initiation of cytokinesis 3 that is about 0-6 hours, about 0-5 hours, e.g. about 0-4 hours, about 0-3 hours, about 0-2 hours, or about 0-1.75 hours, or about 0-1.73 hours. In some embodiments, determining the likelihood that the embryo will reach the blastocyst stage and/or successfully implant into the uterus and/or be euploid can additionally include measuring cellular parameters, including but not limited to: a cell cycle 1 that lasts about 20-27 hours, e.g. about 25-27 hours, time from fertilization to the 5 cell stage that is about 47 hours to about 57 hours, and determining that the embryo does not display an atypical phenotype such as absence of $A1^{cyt}$, AC, AS, immeasurable syngamy or chaotic cleavage.

Examples of direct measurements, any of which alone or in combination are indicative of the likelihood that an embryo will not reach the blastocyst stage and/or implant into the uterus, and/or will be aneuploid include without limitation: (a) a duration of cytokinesis 1 that is more than about 33 minutes, e.g. more than about 35, 40, 45, 50, or 60 minutes; (b) a time interval between the resolution of cytokinesis 1 and the onset of cytokinesis 2 that lasts more than 15 hour, e.g. about 16, 17, 18, 19, or 20 or more hours, or less than 8 hours, e.g. about 7, 5, 4, or 3 or fewer hours; or (c) a time interval between the initiation of cytokinesis 2 and the initiation of cytokinesis 3 that is 6, 7, 8, 9, or 10 or more hours. In some embodiments, determining the likelihood that the embryo will not reach the blastocyst stage and/or implant into the uterus and/or will be aneuploid, can include additionally measuring cellular parameters, including but not limited to: a cell cycle 1 that lasts longer than about 27 hours, e.g. 28, 29, or 30 or more hours, a time interval between fertilization and the 5 cell stage that is less than about 47 hour or more than about 57 hours and/or the detection of $A1^{cyt}$, AS, US, AC and/or chaotic cleavage.

In a preferred embodiment, the methods provide for direct measurement of the duration of P1 and/or P1 phenotypes which alone or in combination with the above identified cellular parameters is indicative of the likelihood that an embryo will not reach the blastocyst stage, and/or become a good quality blastocyst and/or implant into the uterus and/or will be aneuploid. For example, embryos displaying prolonged P1 duration (i.e. the time period between the appearance of the 1st cleavage furrow to completion of the 1st cell division) of ≥0.5 hr and/or one or more abnormal P1 phenotypes ($A1^{cyt}$) (including, e.g., membrane ruffling, oolemma ruffling and/or formation of pseudo cleavage furrows prior to the initiation and/or completion of the first cytokinesis (P1)) are less likely to reach the blastocyst stage or implant into the uterus and/or are more likely to be aneuploid. Similarly, embryos that display AS as evidenced by disordered PN movement within the cytoplasm without prompt dispersion of nuclear envelopes, and/or active oolema movement before the dispersion of the nuclear envelopes and/or a short time period between syngamy and the beginning of the first cytokinesis ($P_{syn}$), wherein short is less than about 1 hour, or less than about 55 minutes, or less than about 50 minutes, or less than about 45 minutes or less than about 40 minutes or less than about 35 minutes, or less than about 30 minutes or less than about 25 minutes or less than about 20 minutes or less than about 15 minutes or less than about 10 minutes or less than about 5 minutes are less likely to reach the blastocyst stage or implant into the uterus and/or are more likely to be aneuploid. Similarly, embryos that display immeasurable syngamy, abnormal cleavage (AC) and/or chaotic cleavage are also less likely to reach blastocyst stage, and/or become good quality blastocysts and/or implant into the uterus and/or are more likely to be aneuploid.

In some embodiments, the cell parameter measurement is employed by comparing it to a cell parameter measurement from a reference, or control, embryo, and using the result of this comparison to provide a determination of the likelihood of the embryo to reach or not reach the blastocyst stage, and/or become a good quality blastocyst and/or implant into the uterus and/or be euploid. The terms "reference" and "control" as used herein mean a standardized embryo or cell to be used to interpret the cell parameter measurements of a given embryo and assign a determination of the likelihood of the embryo to reach or not reach the blastocyst stage, and/or become a good quality blastocyst and/or implant into the uterus and/or be euploid or aneuploid. The reference or control may be an embryo that is known to have a desired phenotype, e.g., likely to reach the blastocyst stage, and/or become a good quality blastocyst and/or implant into the uterus and/or be euploid, and therefore may be a positive reference or control embryo. Alternatively, the reference/control embryo may be an embryo known to not have the desired phenotype, and therefore be a negative reference/control embryo.

In certain embodiments, cellular parameters are first employed to determine whether an embryo will be likely to reach the blastocyst stage, and/or become a good quality blastocyst and/or implant into a uterus and/or be euploid. In such embodiments, embryos that fall within one or more of the above referenced cellular parameter time frames (e.g. a time between cytokinesis 1 and cytokinesis 2 of about 7.8 to about 14.3 hours and/or a time between cytokinesis 2 and cytokinesis 3 of about 0 to about 5.8 hours) is selected to have good developmental potential and/or be euploid. These embryos are then analyzed to determine if they have a normal P1 duration and/or P1 phenotypes, and/or normal syngamy phenotype and/or normal cleavage phenotypes (i.e. absence of AC and/or chaotic cleavage). Embryos previously selected to have good developmental potential/be euploid are deselected when they are determined to have prolonged P1 duration and/or one or more abnormal P1 phenotypes (A1$^{cyt}$) and/or display AS, immeasurable syngamy, AC (e.g. AC1 and/or AC2) and/or chaotic cleavage, thereby selecting for implantation or freezing for potential future implantation, only those embryos that fall within the selection criteria and outside the deselection criteria.

In certain embodiments, the obtained cell parameter measurement(s) is compared to a comparable cell parameter measurement(s) from a single reference/control embryo to obtain information regarding the phenotype of the embryo/cell being assayed. In yet other embodiments, the obtained cell parameter measurement(s) is compared to the comparable cell parameter measurement(s) from two or more different reference/control embryos to obtain more in depth information regarding the phenotype of the assayed embryo/cell. For example, the obtained cell parameter measurements from the embryo(s) being assessed may be compared to both a positive and negative embryo to obtain confirmed information regarding whether the embryo/cell has the phenotype of interest.

As an example, the resolution of cytokinesis 1 and the onset of cytokinesis 2 in normal human embryos is about 8-15 hours, more often about 9-13 hours, with an average value of about 11 +/- 2.1 hours; i.e. 6, 7, or 8 hours, more usually about 9, 10, 11, 12, 13, 14 or up to about 15 hours. A longer or shorter cell cycle 2 in the embryo being assessed as compared to that observed for a normal reference embryo is indicative of the likelihood that the embryo will not reach the blastocyst stage and/or implant into the uterus and/or will be aneuploid. As a second example, the time interval between the initiation of cytokinesis 2 and the initiation of cytokinesis 3, i.e. the synchronicity of the second and third mitosis, in normal human embryos is usually about 0-5 hours, more usually about 0, 1, 2 or 3 hours, with an average time of about 1 +/- 1.6 hours; a longer interval between the completion of cytokinesis 2 and cytokinesis 3 in the embryo being assessed as compared to that observed in a normal reference embryo is indicative of the likelihood that the embryo will not reach the blastocyst stage and/or implant into the uterus and/or will be aneuploid. As a third example, cell cycle 1 in a normal embryo, i.e. from the time of fertilization to the completion of cytokinesis 1, is typically completed in about 20-27 hours, more usually in about 25-27 hours, i.e. about 15, 16, 17, 18, or 19 hours, more usually about 20, 21, 22, 23, or 24 hours, and more usually about 25, 26 or 27 hours. A cell cycle 1 that is longer in the embryo being assessed as compared to that observed for a normal reference embryo is indicative of the likelihood that the embryo will not reach the blastocyst stage and/or implant into the uterus and/or will be aneuploid. As a fourth example, embryos that display A1$^{cyt}$, AS, immeasurable syngamy, AC and/or chaotic cleavage are less likely to reach the blastocyst stage and/or develop into good quality blastocysts and/or are more likely to be aneuploid. Examples may be derived from empirical data, e.g. by observing one or more reference embryos alongside the embryo to be assessed. Any reference embryo may be employed, e.g. a normal reference that is likely to reach the blastocyst stage, and/or develop into a good quality blastocyst and/or implant into the uterus and/or be euploid, or an abnormal reference sample that is not likely to reach the blastocyst stage and/or is likely to be aneuploid. In some cases, more than one reference sample may be employed, e.g. both a normal reference sample and an abnormal reference sample may be used.

As discussed above, one or more parameters may be measured and employed to determine the likelihood of reaching the blastocyst stage for an embryo. In some embodiments, a measurement of two parameters may be sufficient to arrive at a determination of the likelihood of reaching the blastocyst stage and/or becoming a good quality blastocyst and/or implant into the uterus and/or be euploid. In some embodiments, it may be desirable to employ measurements of more than two parameters, for example, 3 cellular parameters or 4 or more cellular parameters. In some embodiments, it may be desirable to measure one or more parameters for selecting an embryo with good developmental potential and/or likelihood of being euploid and one or more parameters for deselecting embryos with poor developmental potential and/or with a likelihood of being aneuploid. In certain embodiments, 1 selection parameter and 1 deselection parameter is measured. In another embodiment, 1 selection parameter and 2 deselection parameters are measured. In another embodiment, 1 selection parameter and 3 deselection parameters are measured. In another embodiment, 2 selection parameters and 1 deselection parameter are measured. In another embodiment, 3 selection parameter and 1 deselection parameter are measured. In another embodiment, more than 3 selection parameters and 1 deselection parameter are measured. In another embodiment, 2 selection parameters and 2 deselection parameters are measured. In another embodiment, 2 selection parameters and 3 deselection parameters are measured. In another embodiment, 3 selection parameters and 2 deselection parameters are measured. In another embodiment, more than 3 selection parameters and 2 deselection parameters are measured. In another embodiment, more than 3 selection parameters and 3 deselection parameters are measured.

In certain embodiments, assaying for multiple parameters may be desirable as assaying for multiple parameters may provide for greater sensitivity and specificity. By sensitivity it is meant the proportion of actual positives which are correctly identified as being such. This may be depicted mathematically as:

$$\text{Sensitivity} = \frac{(\text{Number of true positives})}{(\text{Number of true positives} + \text{Number of false negatives})}$$

Thus, in a method in which "positives" are the embryos that have good developmental potential, i.e. that will develop into blastocysts, and/or become a good quality blastocyst and/or implant into the uterus and/or be euploid, and "negatives" are the embryos that have poor developmental potential, i.e. that will not develop into blastocysts nor develop into good quality blastocysts or implant into the uterus and/or will be aneuploid, a sensitivity of 100% means that the test recognizes all embryos that will develop into blastocysts, or become good quality blastocysts or implant in to the uterus as such. In some embodiments, the sensitivity of the assay may be about 70%, 80%, 90%, 95%, 98% or more, e.g. 100%. By specificity it is meant the proportion of "negatives" which are correctly identified as such. As discussed above, the term "specificity" when used herein with respect to prediction and/or evaluation methods is used to refer to the ability to predict or evaluate an embryo for determining the likelihood that the embryo will not develop into a blastocyst, nor become a good quality blastocyst or implant into the uterus by assessing, determining, identifying or selecting embryos that are not likely to reach the blastocyst stage and/or become a good quality blastocyst and/or implant into the uterus and/or be euploid. This may be depicted mathematically as:

$$\text{Specificity} = \frac{(\text{Number of true negatives})}{(\text{Number of true negatives} + \text{Number of false positives})}$$

Thus, in a method in which positives are the embryos that are likely to reach the blastocyst stage and/or become good quality blastocysts and/or implant into the uterus and/or be euploid (i.e., that are likely to develop into blastocysts), and negatives are the embryos that are likely not to reach the blastocyst stage (i.e., that are not likely to develop into blastocysts) a specificity of 100% means that the test recognizes all embryos that will not develop into blastocysts, i.e. will arrest prior to the blastocyst stage and/or will be aneuploid. In some embodiments, the specificity can be a "high specificity" of 70%, 72%, 75%, 77%, 80%, 82%, 85%, 88%, 90%, 92%, 95%, 98% or more, e.g. 100%. The use of two parameters provides sensitivity of 40%, 57%, 68%, 62%, 68% and specificity of 86%, 88%, 83%, 83%, 77%, respectively. In other words, in one exemplary embodiment, the methods of the invention are able to correctly identify the number of embryos that are going to develop into blastocysts and/or be euploid at least about 40%-68% of the time (sensitivity), and the number of embryos that are going to arrest before the blastocyst stage at least about 77%-88% of the time (specificity), regardless of the algorithm model employed, and as such the present invention provides a high specificity method for identifying the embryos that will arrest before the blastocyst stage or not develop into good quality blastocysts. In addition, the specified mean values and/or cut-off points may be modified depending upon the data set used to calculate these values as well as the specific application.

In some embodiments, the assessment of an embryo or includes generating a written report that includes the artisan's assessment of the subject embryo, e.g. "assessment/selection/determination of embryos likely and/or not likely to reach the blastocyst stage and/or develop into good quality blastocysts and/or implant into the uterus", an "assessment of chromosomal abnormalities", etc. Thus, a subject method may further include a step of generating or outputting a report providing the results of such an assessment, which report can be provided in the form of an electronic medium (e.g., an electronic display on a computer monitor), or in the form of a tangible medium (e.g., a report printed on paper or other tangible medium).

A "report," as described herein, is an electronic or tangible document which includes report elements that provide information of interest relating to an assessment arrived at by methods of the invention. A subject report can be completely or partially electronically generated. A subject report includes at least an assessment of the likelihood of the subject embryo or to reach the blastocyst stage and/or implant into the uterus, an assessment of the probability of the existence of chromosomal abnormalities, etc. A subject report can further include one or more of: 1) information regarding the testing facility; 2) service provider information; 3) subject data; 4) sample data; 5) a detailed assessment report section, providing information relating to how the assessment was arrived at, e.g. a) cell parameter measurements taken, b) reference values employed, if any; and 6) other features.

The report may include information about the testing facility, which information is relevant to the hospital, clinic, or laboratory in which sample gathering and/or data generation was conducted. Sample gathering can include how the sample was generated, e.g. how it was harvested from a subject, and/or how it was cultured etc. Data generation can include how images were acquired or gene expression profiles were analyzed. This information can include one or more details relating to, for example, the name and location of the testing facility, the identity of the lab technician who conducted the assay and/or who entered the input data, the date and time the assay was conducted and/or analyzed, the location where the sample and/or result data is stored, the lot number of the reagents or culture media (e.g., kit, etc.) used in the assay, and the like. Report fields with this information can generally be populated using information provided by the user.

The report may include information about the service provider, which may be located outside the healthcare facility at which the user is located, or within the healthcare facility. Examples of such information can include the name and location of the service provider, the name of the reviewer, and where necessary or desired the name of the individual who conducted sample preparation and/or data generation. Report fields with this information can generally be populated using data entered by the user, which can be selected from among pre-scripted selections (e.g., using a drop-down menu). Other service provider information in the report can include contact information for technical information about the result and/or about the interpretive report.

The report may include a subject data section, including medical history of subjects from which oocytes or were harvested, patient age, in vitro fertilization cycle characteristics (e.g. fertilization rate, day 3 follicle stimulating hormone (FSH) level), and, when oocytes are harvested, zygote/embryo cohort parameters (e.g. total number of embryos). This subject data may be integrated to improve embryo assessment and/or help determine the optimal number of embryos to transfer. The report may also include administrative subject data (that is, data that are not essential to the assessment of the likelihood of reaching the blastocyst stage) such as information to identify the subject (e.g., name, subject date of birth (DOB), gender, mailing and/or residence address, medical record number (MRN), room and/or bed number in a healthcare facility), insurance information, and the like), the name of the subject's physician or other health professional who ordered the assessment of developmental potential and, if different from the ordering physician, the name of a staff physician who is responsible for the subject's care (e.g., primary care physician).

The report may include a sample data section, which may provide information about the biological sample analyzed in the assessment, such as how the sample was handled (e.g. storage temperature, preparatory protocols) and the date and time collected. Report fields with this information can generally be populated using data entered by the user, some of which may be provided as pre-scripted selections (e.g., using a drop-down menu).

The report may include an assessment report section, which may include information relating to how the assessments/determinations were arrived at as described herein. The interpretive report can include, for example, time-lapse images of the embryo being assessed, and/or gene expression results. The assessment portion of the report can optionally also include a recommendation(s) section. For example, where the results indicate that the embryo is likely to reach the blastocyst stage and/or implant into the uterus, the recommendation can include a recommendation that a limited number of embryos be transplanted into the uterus during fertility treatment as recommended in the art.

It will also be readily appreciated that the reports can include additional elements or modified elements. For example, where electronic, the report can contain hyperlinks which point to internal or external databases which provide more detailed information about selected elements of the report. For example, the patient data element of the report can include a hyperlink to an electronic patient record, or a site for accessing such a patient record, which patient record is maintained in a confidential database. This latter embodiment may be of interest in an in-hospital system or in-clinic setting. When in electronic format, the report is recorded on a suitable physical medium, such as a computer readable medium, e.g., in a computer memory, zip drive, CD, DVD, etc.

It will be readily appreciated that the report can include all or some of the elements above, with the proviso that the report generally includes at least the elements sufficient to provide the analysis requested by the user (e.g., an assessment of the likelihood of reaching the blastocyst stage, and/or develop into good quality blastocysts and/or implant into the uterus).

As discussed above, methods of the invention may be used to assess embryos or cells to determine the likelihood of the embryos to reach the blastocyst stage, and/or develop into good quality blastocysts and/or implant into the uterus and/or be euploid. This determination of the likelihood of the embryos or to reach the blastocyst stage and/or implant into the uterus and/or be euploid may be used to guide clinical decisions and/or actions. For example, in order to increase pregnancy rates, clinicians often transfer multiple embryos into patients, potentially resulting in multiple pregnancies that pose health risks to both the mother and fetuses. Using results obtained from the methods of the invention, the likelihood of reaching the blastocyst stage, and/or develop into good quality blastocysts and/or implant into the uterus and/or be euploid can be determined for embryos being transferred. As the embryos that are likely to reach the blastocyst stage, and/or develop into good quality blastocysts and/or implant into the uterus and/or be euploid are more likely to develop into fetuses, the determination of the likelihood of the embryo to reach the blastocyst stage , and/or develop into good quality blastocysts and/or implant into the uterus and/or be euploid prior to transplantation allows the practitioner to decide how many embryos to transfer so as to maximize the chance of success of a full term pregnancy while minimizing risk.

Assessments made by following methods of the invention may also find use in ranking embryos or in a group of embryos or for their likelihood that the embryos or will reach the blastocyst stage as well as for the quality of the blastocyst that will be achieved (e.g., in some embodiments this would include the likelihood of implanting into the uterus). For example, in some instances, multiple embryos may be capable of developing into blastocysts, i.e. multiple embryos are likely to reach the blastocyst stage. However, some embryos will be more likely to achieve the blastocyst stage, i.e. they will have better likelihood to reach the blastocyst stage, or better likelihood to develop into good quality blastocyst, or better likelihood to implant into the uterus than other embryos. In such cases, methods of the invention may be used to rank the embryos in the group. In such methods, one or more cellular parameters for each embryo is measured to arrive at a cell parameter measurement for each embryo. The one or more cell parameter measurements from each of the embryos are then employed to determine the likelihood of the embryos relative to one another to reach the blastocyst stage and/or to implant into the uterus. In some embodiments, the cell parameter measurements from each of the embryos or are employed by comparing them directly to one another to determine the likelihood of reaching the blastocyst stage and/or implant into the uterus. In some embodiments, the cell parameter measurements from each of the embryos are employed by comparing the cell parameter measurements to a cell parameter measurement from a reference embryo to determine likelihood of reaching the blastocyst stage and/or implant into the uterus for each embryo, and then comparing the determination of the likelihood of reaching the blastocyst stage and/or implant into the uterus for each embryo to determine the likelihood of reaching the blastocyst stage and/or implant into the uterus of the embryos or relative to one another.

In this way, a practitioner assessing, for example, multiple zygotes/embryos, can choose only the best quality embryos, i.e. those with the best likelihood of reaching the blastocyst stage and/or implant into the uterus, to transfer so as to maximize the chance of success of a full term pregnancy while minimizing risk.

Also provided are reagents, devices and kits thereof for practicing one or more of the above-described methods. The subject reagents, devices and kits thereof may vary greatly. Reagents and devices of interest include those mentioned above with respect to the methods of measuring any of the aforementioned cellular parameters, where such reagents may include culture plates, culture media, microscopes, imaging software, imaging analysis software, nucleic acid primers, arrays of nucleic acid probes, antibodies, signal producing system reagents, etc., depending on the particular measuring protocol to be performed.

In addition to the above components, the subject kits will further include instructions for practicing the subject methods. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g., diskette, CD, etc., on which the information has been recorded. Yet another means that may be present is a website address which may be used via the internet to access the information at a removed site. Any convenient means may be present in the kits.

Some of the methods described above require the ability to observe embryo development via time-lapse imaging. This can be achieved using a system comprised of a miniature, multi-channel microscope array that can fit inside a standard incubator. This allows multiple samples to be imaged quickly and simultaneously without having to physically move the dishes. One illustrative prototype, shown in Fig. 22 of US Patent No. 7,963,906 (*See also* PCT/2011/053537), consists of a 3-channel microscope array with darkfield illumination, although other types of illumination could be used. By "three channel," it is meant that there are three independent microscopes imaging three distinct culture dishes simultaneously. A stepper motor is used to adjust the focal position for focusing or acquiring 3D image stacks. White-light LEDs are used for illumination, although we have observed that for human embryos, using red or near-infrared (IR) LEDs can improve the contrast ratio between cell membranes and the inner portions of the cells. This improved contrast ratio can help with both manual and automated image analysis. In addition, moving to the infrared region can reduce phototoxicity to the samples. Images are captured by low-cost, high-resolution webcams, but other types of cameras may be used.

As shown in Fig. 22 of US Patent No. 7,963,906 (*See also* PCT/2011/053537), each microscope of the prototype system described above is used to image a culture dish which may contain anywhere from 1-30 embryos. The microscope collects light from a white light LED connected to a heat sink to help dissipate any heat generated by the LED, which is very small for brief exposure times. The light passes through a conventional dark field patch for stopping direct light, through a condenser lens and onto a specimen labeled "petri dish," which is a culture dish holding the embryos being cultured and studied. The culture dish may have wells that help maintain the order of the embryos and keep them from moving while the dish is being carried to and from the incubator. The wells can be spaced close enough together so that embryos can share the same media drop. The scattered light is then passed through a microscope objective, then through an achromat doublet, and onto a CMOS sensor. The CMOS sensor acts as a digital camera and is connected to a computer for image analysis and tracking as described above.

This design is easily scalable to provide significantly more channels and different illumination techniques, and can be modified to accommodate fluidic devices for feeding the samples. In addition, the design can be integrated with a feedback control system, where culture conditions such as temperature, CO2 (to control pH), and media are optimized in real-time based on feedback and from the imaging data. This system was used to acquire time-lapse videos of human embryo development, which has utility in determining embryo viability for in vitro fertilization (IVF) procedures. Other applications include stem cell therapy, drug screening, and tissue engineering.

In one embodiment of the device, illumination is provided by a Luxeon white light-emitting diode (LED) mounted on an aluminum heat sink and powered by a BuckPuck current regulated driver. Light from the LED is passed through a collimating lens. The collimated light then passes through a custom laser-machined patch stop, as shown in Fig. 22 of US Patent No. 7,963,906, and focused into a hollow cone of light using an aspheric condenser lens. Light that is directly transmitted through the sample is rejected by the objective, while light that is scattered by the sample is collected. In one embodiment, Olympus objectives with 20X magnification are used, although smaller magnifications can be used to increase the field-of-view, or larger magnifications can be used to increase resolution. The collected light is then passed through an achromat doublet lens (i.e. tube lens) to reduce the effects of chromatic and spherical aberration. Alternatively, the collected light from the imaging objective can be passed through another objective, pointed in the opposing direction, that acts as a replacement to the tube lens. In one configuration, the imaging objective can be a 10X objective, while the tube-lens objective can be a 4X objective. The resulting image is captured by a CMOS sensor with 2 megapixel resolution (1600 x 1200 pixels). Different types of sensors and resolutions can also be used.

For example, Fig. 23A of US Patent No. 7,963,906 (*See also* PCT/2011/053537) shows a schematic of the multi-channel microscope array having 3 identical microscopes. All optical components are mounted in lens tubes. In operation of the array system, Petri dishes are loaded on acrylic platforms that are mounted on manual 2-axis tilt stages, which allow adjustment of the image plane relative to the optical axis. These stages are fixed to the base of the microscope and do not move after the initial alignment. The illumination modules, consisting of the LEDs, collimator lenses, patch stops, and condenser lenses, are mounted on manual xyz stages for positioning and focusing the illumination light. The imaging modules, consisting of the objectives, achromat lenses, and CMOS sensors, are also mounted on manual xyz stages for positioning the field-of-view and focusing the objectives. All 3 of the imaging modules are attached to linear slides and supported by a single lever arm, which is actuated using a stepper motor. This allows for computer-controlled focusing and automatic capture of image-stacks. Other methods of automatic focusing as well as actuation can be used.

The microscope array was placed inside a standard incubator, as shown in, for example, Fig. 23B of US Patent No. 7,963,906 (*See also* PCT/2011/053537). The CMOS image sensors are connected via USB connection to a single hub located inside the incubator, which is routed to an external PC along with other communication and power lines. All electrical cables exit the incubator through the center of a rubber stopper sealed with silicone glue.

The above described microscope array, or one similar, can be used to record time-lapse images of early human embryo development and documented growth from zygote through blastocyst stages. In some embodiments, images can be captured every 5 minutes with roughly 1 second of low-light exposure per image. The total amount of light received by the samples can be equivalent to 52 seconds of continuous exposure, similar to the total level experienced in an IVF clinic during handling. The 1 second duration of light exposure per image can in some embodiments be reduced. Prior to working with the human embryos, we performed extensive control experiments with mouse pre-implantation embryos to ensure that both the blastocyst formation rate and gene expression patterns were not affected by the imaging process.

Individual embryos can be followed over time, even though their positions in the photographic field shifted as the embryos underwent a media change, in some cases the media was changed at day 3. The use of sequential media may be needed to meet the stage-specific requirements of the developing embryos. During media change, the embryos were removed from the imaging station for a few minutes and transferred to new petri dishes. The issue of tracking embryo identity can be mitigated by using wells to help arrange the embryos in a particular order.

When transferring the petri dishes between different stations, the embryos can sometimes move around, thereby making it difficult to keep track of embryo identity. This poses a challenge when time-lapse imaging is performed on one station, and the embryos are subsequently moved to a second station for embryo selection and transfer. One method is to culture embryos in individual petri dishes. However, this requires each embryo to have its own media drop. In a typical IVF procedure, it is usually desirable to culture all of a patient's embryos on the same petri dish and in the same media drop. To address this problem, we have designed a custom petri dish with micro-wells. This keeps the embryos from moving around and maintains their arrangement on the petri dish when transferred to and from the incubator or imaging stations. In addition, the wells are small enough and spaced closely together such that they can share the same media drop and all be viewed simultaneously by the same microscope. The bottom surface of each micro-well has an optical quality finish. For example, Fig. 27A in US Patent No. 7,963,906 (*See also* PCT/2011/053537) shows a drawing with dimensions for one exemplary embodiment. In this version, there are 25 micro-wells spaced closely together within a 1.7 x 1.7 mm field-of-view. Fig. 27B of US Patent No. 7,963,906 (*See also* PCT/2011/053537) shows a 3D-view of the micro-wells, which are recessed approximately 100 microns into the dish surface. Fiducial markers, including letters, numbers, and other markings, are included on the dish to help with identification. All references cited herein, including patents, patent applications, manuscripts and the like are specifically and fully incorporated by reference in their entireties.

## EXAMPLES

The following examples are put forth so as to provide those of ordinary skill in the art with a disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

## EXAMPLE 1

A multisite retrospective cohort study was undertaken using image data collected from 651 embryos from 67 patients from five clinics in a 16 month period. Patient embryos were imaged using the Eeva™ Test (Auxogyn, Inc.), a time-lapse imaging system developed for blastocyst prediction which performs time-lapse analysis of key cell division timings.

All imaged embryos were identified as having 2 pronuclei (PN) before being placed in a multi-well Eeva dish that allows embryos to be tracked individually while sharing a single drop of culture media. A fertilization check was performed according to each clinic's standard protocol. All 2PN embryos were transferred to the Eeva dish immediately after fertilization status was assessed, and the dish was placed on the Eeva scope in the incubator. To maintain a continuous and uninterrupted imaging process from Day 1 through Day 3, no media changes or dish removal from the incubator were permitted. On Day 3, imaging was stopped just before routine embryo grading was performed. All embryos were tracked individually to maintain their identities. Embryo grading, selection and transfer (on Days 3 or 5), were performed according to the standard operating procedures of each individual clinic.

Embryo development outcome was measured by overall morphology grade and blastocyst formation rate. The overall embryo grade was determined using cleavage stage and blastocyst stage morphological grading as defined by the Society for Assisted Reproductive Technology (SART) (Racowsky, C., et al., Fertil Steril, 2010. 94(3): p. 1152-3; Vernon, M., et al., Fertil Steril, 2011. 95(8): p. 2761-3). Further discrimination among the Day 3 embryos with 6-10 cells was included in the analysis to focus on the top quality embryos with ≤10% fragmentation. Implantation was confirmed by ultrasound showing evidence of intrauterine fetal heart motion at approximately 6-8 weeks gestational age. Known implantation included data in which the embryo implantation status was confirmed, e.g., number of gestational sacs matched the number of transferred embryos.

Mean values were compared using a *t*-test using SAS. Associations between presence or absence of atypical phenotypes and embryo quality and development potential were examined using a chi-squared test or Fisher's Exact test to assess statistical significance, where $p < 0.05$ was considered to be statistically significant.

Embryo videos were reviewed for 1st cytokinesis (P1) phenotype and duration. "Abnormal phenotype" or "A1$^{cyt}$" was defined as oolemma ruffling and/or formation of pseudo cleavage furrows. P1 duration was defined as the time from actual furrow formation to the time point when the new daughter cells are completely separated by confluent cell membranes. Two additional sub-groups were then created: embryos exhibiting A1$^{cyt}$ with prolonged P1 (P1≥0.5 hours) or shorter P1 (P1<0.5 hours). The control group was composed of embryos that did not exhibit either A1$^{cyt}$ or prolonged P1.

The overall prevalence of A1$^{cyt}$ was 30.7% among all embryos reviewed and 88.0% of the patients had A1$^{cyt}$ embryos (59/67). Compared to control embryos (without A1$^{cyt}$), embryos exhibiting A1$^{cyt}$ had lower rate of good morphology embryos on Day 3 (6-10 cells and ≤10% fragmentation, 40.7% vs. 62.9%, p<0.0001), higher rate of embryos with high fragmentation (>25% fragmentation, 16.1% (19/118) vs. 6.7%) (23/345), p<0,001), fewer cleavage embryos with overall grade good or fair, (79.7% vs. 90.7%, p=0.001), and lower blastocyst formation rate (21.7% vs. 44.6%, p<0.0001) (Table 2). However, both groups formed similar rates of good or fair quality blastocysts (69.2% vs. 52.3%, p=0.1) and exhibited distinct but statistically significant differences in implantation rate (6.2% vs. 16.5%, p=0.1).

Table 2. Abnormal First Cytokinesis (A1$^{cyt}$): Embryo Quality and Developmental Potential for Day 3 and Day 5 Embryos.

| Abnormal First Cytokinesis (A1$^{cyt}$) | Day 3 6-10 cells ≤10% frag | Day 3 Overall Grade Good/Fair | Blastocyst Formation Rate | Blastocyst Overall Grade Good/Fair | Transferred or Frozen Embryos | Known Implantation Data |
|---|---|---|---|---|---|---|
| Control: Without A1$^{cyt}$ (n=443) | 62.9% (217/345) | 90.7% (313/345) | 44.6% (131/294) | 52.3% (69/131) | 48.5% (215/443) | 16.5% (15/91) |
| With A1$^{cyt}$ (n=196) | 40.7% (48/119) | 79.7% (94/119) | 21.67% (26/120) | 69.2% (18/26) | 34.2% (67/196) | 6.2% (2/32) |
| p-value | <0.0001 | <0.001 | <0.0001 | 0.1 | 0.0005 | 0.1 |

A subset of 53.1% embryos exhibiting A1$^{cyt}$ also exhibited prolonged P1 timing (0.5±0.8 vs. 1.8±3.3 hours, p<0.0001). Compared to control embryos, the subgroup of embryos with P1≥0.5 hours had even lower blastocyst formation rate (9.2% vs. 44.6%, p<0.0001), and none of the embryos with A1$^{cyt}$ and P1≥0.5 hours implanted. To assess this subgroup further, embryos with A1$^{cyt}$ were compared based on P1 (Table 3). Compared to A1$^{cyt}$ embryos with shorter P1, A1$^{cyt}$ embryos with prolonged P1 had poorer morphology on Day 3 (6-10 cells and ≤10% fragmentation, 18.5% vs. 58.5%, p<0.0001), higher rate of embryos with high fragmentation (>25% fragmentation, 26.4% vs. 7.7%, p<0.0001), and lower blastocyst formation rate (9.2%) vs. 36.4%, p<0.0003). The difference in implantation rate was notable but not statistically significant (0.0% vs. 11.8%, p=0.3). When considering the number of transferred or frozen embryos, the A1$^{cyt}$ phenotype group had 34.2%, while the control group had 48.5% (p=0.0005). The majority of transferred or frozen embryos with A1$^{cyt}$ had shorter P1 times (65.7%, 44/67).

Table 3. Abnormal First Cytokinesis (A1$^{cyt}$) and P1: Embryo Quality and Developmental Potential for Day 3 and Day 5 Embryos.

| Abnormal First Cytokinesis (A1$^{cyt}$) | Day 3 6-10 cells ≤10% frag | Day 3 Overall Grade Good/Fair | Blastocyst Formation Rate | Blastocyst Overall Grade Good/Fair | Transferred or Frozen Embryos | Known Implantation Data |
|---|---|---|---|---|---|---|
| With A1$^{cyt}$ and P1≥0.5 (n=104) | 18.5% (10/54) | 64.8% (35/54) | 9.2% (6/65) | 66.7% (4/6) | 22.1% (23/104) | 0.0% (0/14) |
| With A1$^{cyt}$ and P1<0.5 (n=92) | 58.5% (38/65) | 90.8% (59/65) | 36.4% (20/55) | 70.0% (14/20) | 47.8% (44/92) | 11.8% (2/17) |
| p-value | <0.0001 | <0.001 | 0.0003 | 0.9 | 0.0002 | 0.3 |

Embryos exhibiting A1$^{cyt}$ phenotypes represent 31% of the embryo population and have significantly lower developmental potential. Additionally, approximately half of the embryos exhibiting A1$^{cyt}$ also exhibited a prolonged first cytokinesis timing (P1≥0.5 hours), which was associated with lower rates of blastocyst formation compared to A1$^{cyt}$ embryos with shorter first cytokinesis timing (P1<0.5 hours).

The combination of A1$^{cyt}$ phenotype and the timing of the first cytokinesis may be used together to finely discriminate those embryos with low developmental competence. A1$^{cyt}$ embryos with P1≥0.5 hours had the lowest blastocyst formation of all subgroups evaluated. Importantly, many A1$^{cyt}$ embryos in both timing groups have good morphology on Day 3, and the A1$^{cyt}$ embryos that are able to develop to Day 5 are mostly good quality blastocysts that have the potential to be selected for transfer. Fewer numbers of embryos with A1$^{cyt}$ were selected for transfer, and a very low implantation rate was observed. Since many of these embryos have good morphology at the cleavage stage, using time-lapse to detect abnormal and prolonged 1st cytokinesis phenotypes may improve the success of embryo selection.

The AC phenotype was defined as embryos producing more than 2 cells during a single cell division event. Two independent types of AC phenotypes were evaluated. AC1 phenotypes were recorded when embryos exhibited a first cleavage yielding more than two blastomeres, and AC2 phenotypes were recorded when embryos exhibited a daughter cell cleavage yielding more than two blastomeres. The control group was composed of embryos that had an appropriate first cleavage yielding two blastomeres and appropriate daughter cell cleavages, i.e., each yielding two blastomeres.

The overall prevalence of AC embryos was 18.0% among all embryos reviewed (AC1: 35/639 8.3%; AC2: 39/639 9.2%; Both AC1 and AC2: 1/639 0.5%) and 73.1% of the patients had AC embryos (49/67). Both groups, control (without AC) and AC, had similar rates of cleavage embryos with overall grade good or fair (86.3% vs. 88%), p=0.4), as well as similar rates of embryos with high fragmentation (>25% fragmentation, 9.9% (8/81) vs. 8.9% (34/383), p=0.6) (Table 4). However, the AC group had significantly fewer good quality embryos on day 3 (6-10 cells and ≤10% fragmentation, 46.9% vs. 59.3% for control, p=0.4). Among day 3 embryo transfers, the incidence of AC embryos was 28.6%, and the majority of the embryos exhibited AC2, 19.0% (20/105), followed by 8.6% of embryos with AC1 (9/105) and 1.0% of AC1 and AC2 (1/105). Importantly, compared to the control group, AC embryos had lower blastocyst formation rate (11.7% vs. 43.1%, p<0.0001) and showed a trend towards lower implantation rate (3.7% vs. 18.0%, p=0.05), but showed no difference regarding percentage of good or fair blastocysts (62.5% vs. 55%, p=0.7). Regarding the number of transferred or frozen embryos, the control group had 44.8% while the AC group had 37.4% (p=0.1).

Table 4. Abnormal Cleavage (AC): Embryo Quality and Developmental Potential for Day 3 and Day 5 Embryos.

| Abnormal Cleavage (AC) | Day 3 6-10 cells ≤10% frag | Day 3 Overall Grade Good/ Fair | Blastocyst Formation Rate | Blastocyst Overall Grade Good/Fair | Transferred or Frozen Embryos | Known Implantation Data |
|---|---|---|---|---|---|---|
| Control: Without AC (n=524) | 59.3% (227/383) | 88% (337/383) | 43.1% (149-346) | 55.0% (82/149) | 44.8% (239/524) | 18.0% (19/105) |
| With AC (n=115) | 46.9% (38/81) | 86.4% (70/81) | 11.7% (8/68) | 62.5% (5/8) | 37.4% (43/115) | 3.7% (1/27) |
| p-value | 0.04 | 0.4 | <0.0001 | 0.7 | 0.1 | 0.05 |

The present study characterized both AC1 and AC2 phenotypes in the total embryo population, in good morphology embryos, and in embryos that are selected for transfer. Among the 142 embryos transferred on day 3 or day 5, up to 21.1% exhibited at least one AC. More specifically, 6.3% transferred embryos exhibited AC1 (9/142) and 14.0% exhibited AC2 (20/142). The incidence of AC embryos was enriched to 28.6% among day 3 transferred embryos (AC1: 9/105 8.6%; AC2: 20/105 19.0%; Both AC1 and AC2: 1/105 1.0%), and further evaluation revealed that this high AC rate may be explained by the presence of high quality (near perfect symmetry, low fragmentation) AC embryos on day 3. Notably, the only AC embryo transferred on Day 5 was an AC2 embryo that ultimately implanted successfully, whereas no AC1 embryos implanted. AC2 embryos further seemed to have a significantly higher blastocyst rate than AC1 embryos, suggesting that subsequent abnormal cleavage events beyond the first cell division may represent a milder abnormal phenotype where the embryo could potentially have fewer chromosomally abnormal cells or could express mosaicism compatible with embryo development potential and implantation. Further evaluation indicated that the prevalence of AC in patient cohorts (0% vs 1-25% vs. ≥ 25%) was inversely correlated with clinical pregnancy (50% vs. 31.3% vs. 11.1%); patients with a higher frequency of AC in their cohort had statistically lower clinical pregnancy rate (p<0.05). Taken together, these data clearly show that AC embryos reach the blastocyst stage at a much lower rate and display a much lower implantation/pregnancy rate than embryos that do not display AC. These novel cleavage parameters provide for an early indicator of embryos with low developmental potential. Thus, these parameters may be used alone, or in combination with previously described parameters, such as those described in US Patent Nos. 7,963,906; 8,323,177, and 8,337,387 and PCT Appl. No.: WO 2012/163363 to select embryos that are most likely to reach blastocyst and/or implant into the uterus and deselect embryos that are likely to not reach blastocyst and/or implant into the uterus.

Thus, these parameters (i.e. A1$^{cyt}$ phenotypes and/or prolonged P1 duration (P1≥0.5 hrs and/or AC)) may be used alone, or in combination with previously described parameters, such as those described in US Patent Nos. 7,963,906; 8,323,177, and 8,337,387 and PCT Appl. No.: WO 2012/163363 to select embryos that are most likely to reach blastocyst and/or implant into the uterus and deselect embryos that are likely to not reach blastocyst and/or implant into the uterus.

EXAMPLE 2

Further analysis of the retrospective cohort study described in the previous example was performed to evaluate chaotic cleavage.

The chaotic cleavage phenotype was defined by the appearance of disordered cleavage behavior by the 4-cell stage. Chaotic cleavage is visualized using time-lapse microscopy when the first cell divisions are erratic and frequently result in uneven-sized blastomeres and/or fragments. The control group for this phenotype was composed of embryos exhibiting orderly cleavage behavior with clear cell divisions.

The overall prevalence of chaotic cleavage was 15% among all embryos reviewed and 58.2% of the patients had at least one chaotic cleavage embryo (39/67). Compared to the control group (without chaotic cleavage), embryos with chaotic cleavage had poorer morphology on day 3 (6-10 cells and ≤ 10% fragmentation, 3.7% vs. 64.1% p<0.0001), a higher rate of highly fragmented embryos (>25% fragmentation, 61.5% (59/96) vs. 9.6% (52/543), P>0.0001), fewer cleavage stage embryos with an overall grade of good or fair (35.2% vs. 94.6% p<0.0001) and a lower blastocyst formation rate (14.1% vs 42.3%, p<0.001). (Table 5) Both groups presented similar percentages of good or fair blastocysts (55.6% vs. 55.4%, P=0.9). When considering the number of transferred or frozen embryos, the chaotic cleavage group had 13.5% while the control group had 49.5% (p<0.001). Regarding implantation, none of the embryos that displayed chaotic cleavage successfully implanted, while 18.2% of control embryos implanted.

Table 5: Chaotic Cleavage. Comparison of different outcomes between control group (without chaotic cleavage) and embryos with chaotic cleavage

| Chaotic Cleavage | Day 3, 6-10 cells (n=464) | | Blastocyst formation rate (n=414) | Blastocyst overall grade good/fair (n=157) | Transferred or frozen embryos (n=639) | Known implantation data (n=139) |
|---|---|---|---|---|---|---|
| | Overall grade good/fair | ≤10% frag. | | | | |
| Control (no chaotic cleavage) | 94.6% (388/410) | 64.1% (263/410) | 43.3% (148/350) | 55.4% (82/148) | 49.5% (269/543) | 18.2% (24/132) |
| With chaotic cleavage | 35.2% (19/54) | 3.7% (2/54) | 14.0% (9/64) | 55.6% (5/9) | 13.5% 13/96) | 0.0% (0/7) |
| P-value | <0.0001 | <0.0001 | <0.0001 | 0.99 | <0.001 | 0.3 |

Taken together, in our review of atypical embryo phenotypes, we consistently observed a unique pattern in which embryos exhibited erratic cleavage patters, constant movement of the blastomere membranes and frequent fragmentation. This dynamic phenotype usually resulted in asymmetric blastomeres and/or fragments often making it difficult to distinguish the actual cell divisions and resulting cell count. Until now, the chaotic cleavage phenotype has not been described for human embryos.

Most chaotic cleavage embryos (86.5%) were not considered candidates for transfer or freezing when better embryos were available, presumably because highly fragmented embryos are usually the last choice during embryo selection. However, a small percentage of embryos exhibiting chaotic cleavage (35.2%) show low fragmentation patterns and were assigned an overall good or fair morphology grade on day 3. Further, although only 9 out of 64 (14.1%) chaotic cleavage embryos developed into blastocysts, most of these blastocysts were graded as having good or fair morphology. However, of the seven chaotic cleavage embryos that were actually transferred, none of them implanted. Therefore, based on the lower day 3 quality and lower blastocyst formation and quality, this phenotype can be used as a deselection criterion to improve successful assessment. The chaotic cleavage phenotype may also be important to assess since the vast majority of time-lapse parameters associated with clinical outcomes currently being used and investigated are dependent upon the recognition of exact timing of cell division(s).

EXAMPLE 3

Further analysis of the retrospective cohort study described in the previous example was performed to evaluate the A1$^{cyt}$, and or AC and/or chaotic cleavage parameters in combination with other atypical phenotype parameters. Using the data from the 67 patients and 639 embryo movies, a embryos with one ore more atypical phenotypes were analyzed.

The atypical phenotypes examined were abnormal cleavage (AC), abnormal syngamy (AS), abnormal first cytokinesis (A1$^{cyt}$), and chaotic cleavage. The A1$^{cyt}$ and AC phenotypes were defined as described in Example 1. The chaotic cleavage phenotype was defined in Example 2. The AS phenotype was defined as embryos exhibiting disordered movement within the cytoplasm without prompt dispersion of nuclear envelopes.

The overall prevalence of embryos exhibiting one or more atypical phenotype was 54.2% among all embryos reviewed and prevalent in 98.5% (66/67) among all patient cases. Compared to the control group (without any atypical phenotype), embryos with at least one atypical phenotype tended to have fewer good quality embryos on Day 3 (6-10 cells and ≤10% fragmentation, 46.2% vs. 68.4%, p<0.0001), higher rate of embryos highly fragmented (>25% fragmentation, 28.4% (100/352) vs. 5.74% (17/297) p<0.0001) and fewer cleavage embryos with overall grade good or fair (80.3% vs. 94.7%, p<0.0001) (Table 4). Both groups presented similar blastocyst formation rate (52.1% vs. 56.9%, p=0.6) and similar percentages of good or fair quality blastocysts (52.1% vs. 56.9%, p=0.6), but had statistically significant differences in the number of transferred or frozen embryos (34.7% vs. 53.9%, p<0.0001). Embryos with at least one phenotype also had a statistically significant lower implantation rate (8.6% vs. 21.9%, p=0.02).

Table 6. Embryos displaying one or more atypical phenotype: Embryo Quality and Developmental Potential for Day 3 and Day 5 Embryos.

| One or more atypical phenotypes | Day 3 6-10 cells ≤10% frag | Day 3 Overall Grade Good/Fair | Blastocyst Formation Rate | Blastocyst Overall Grade Good/Fair | Transferred or Frozen Embryos | Known Implantation Data |
|---|---|---|---|---|---|---|
| Control: Without any atypical phenotype (n=297) | 67.8% (166/245) | 94.7% (232/245) | 53.7% (109/203) | 56.9% (62/109) | 53.9% (160/297) | 21.9% (16/64) |
| Without one or more atypical phenotypes (n=352) | 45.4% (99/218) | 80.3% (175/218) | 22.4% (48/214) | 52.1% (25/48) | 34.7% (122/352) | 8.6% (5/58) |
| p-value | <0.0001 | <0.0001 | <0.0001 | 0.6 | <0.0001 | 0.02 |

The group of embryos displaying one or more atypical phenotypes was statistically significantly different than the control group for most outcomes. 5 in every 10 embryos showed at least one atypical phenotype: 18.8% of the embryos showed at least 2 phenotypes (122/649), 6.5% showed 3 phenotypes (42/649) and 1.1% showed 4 phenotypes (7/649). This extraordinarily high prevalence within embryo cohorts suggests that tools to deselect these dynamic phenomena are urgently needed to increase the chances of selecting a competent embryo, particularly as this study has demonstrated that many embryos exhibiting normal phenotypes have good conventional morphology on day 3 and day 5.

**Claims**

1.    A method for deselecting one or more human embryos with poor developmental potential comprising:

   (A) in vitro culturing one or more embryos under conditions sufficient for embryo development;
   (B) time lapse imaging said one or more embryos for a time period sufficient to measure at least one cell division; and
   (C) deselecting an embryo with poor developmental potential when the embryo displays an abnormal phenotype at the second cleavage (AC2)

   thereby deselecting an embryo with poor developmental potential, wherein the embryo with poor developmental potential is not likely to reach the blastocyst stage or successfully implant into the uterus.

2.    The method of claim 1 comprising deselecting an embryo when the embryo displays an abnormal phenotype at the second cleavage (AC2) and the first cleavage (AC1).

3.    The method of claim 1 wherein deselection comprises choosing to not implant the embryo determined to have poor developmental potential into the uterus.

4.    The method of claim 1 further comprising measuring one or more cellular parameters selected from the group consisting of:

(a) the duration of the first cytokinesis;
(b) the time between the first and second mitosis;
(c) the time between the second and third mitosis;
(d) the time interval between cytokinesis 1 and cytokinesis 2;
(e) the time interval between cytokinesis 2 and cytokinesis 3;
(f) the time interval between fertilization and the 5 cell stage;
(g) the duration of the first cell cycle; and
(h) the time interval between syngamy and the first cytokinesis.

5. The method of claim 1 wherein said one or more embryos are produced by fertilization of oocytes in vitro and optionally wherein said oocytes are matured in vitro and optionally wherein said oocytes matured in vitro are supplemented with growth factors.

6. The method of claim 1 wherein said one or more embryos have not been frozen prior to culturing.

7. The method of claim 1 wherein said one or more embryos have been frozen prior to culturing.

8. The method of claim 1 wherein the deselecting an embryo with poor developmental potential is automated.

9. The method of claim 1 wherein said time lapse imaging acquires images that are digitally stored.

10. The method of claim 1 wherein said time lapse imaging employs darkfield illumination.

11. The method of claim 1 wherein said one or more human embryos are placed in a culture dish prior to culturing under conditions sufficient for embryo development.

12. The method of claim 11 wherein said culture dish comprises a plurality of microwells and optionally wherein said culture dish comprises from 1 to about 30 microwells.

13. The method of claim 12 wherein one or more human embryos is placed within a microwell prior to culturing under conditions sufficient for embryo development.

14. The method of claim 1 wherein the measuring is carried out at an imaging station.

**Patentansprüche**

1. Verfahren zur Deselektierung eines oder mehrerer menschlicher Embryonen mit schlechtem Entwicklungspotenzial, umfassend:

   (A) *in vitro* Kultivierung eines oder mehrerer Embryonen unter Bedingungen, die für die Embryonenentwicklung ausreichend sind;
   (B) Zeitrafferaufnahme des einen oder der mehreren Embryonen über einen Zeitraum hinweg, der ausreicht, um mindestens eine Zellteilung zu messen; und
   (C) Deselektieren eines Embryos mit schlechtem Entwicklungspotenzial, wenn der Embryo bei der zweiten Teilung (AC2) einen abnormalen Phänotyp aufweist,

   wodurch ein Embryo mit schlechtem Entwicklungspotenzial deselektiert wird, wobei der Embryo mit schlechtem Entwicklungspotenzial wahrscheinlich das Blastozystenstadium nicht erreicht oder sich erfolgreich in die Gebärmutter einnistet.

2. Verfahren nach Anspruch 1, umfassend die Deselektierung eines Embryos, wenn der Embryo bei der zweiten Teilung (AC2) und der ersten Teilung (AC1) einen abnormalen Phänotyp aufweist.

3. Verfahren nach Anspruch 1, wobei die Deselektierung das Auswählen umfasst, den Embryo, bei dem ein schlechtes Entwicklungspotenzial bestimmt wurde, einzunisten.

4. Verfahren nach Anspruch 1, weiter umfassend das Messen eines oder mehrerer Zellparameter, ausgewählt aus der Gruppe bestehend aus:

(a) die Dauer der ersten Zytokinese;
(b) die Zeit zwischen der ersten und zweiten Mitose;
(c) die Zeit zwischen der zweiten und dritten Mitose;
(d) das Zeitintervall zwischen Zytokinese 1 und Zytokinese 2;
(e) das Zeitintervall zwischen Zytokinese 2 und Zytokinese 3;
(f) das Zeitintervall zwischen der Befruchtung und dem Fünfzellstadium;
(g) die Dauer des ersten Zellzyklus; und
(h) das Zeitintervall zwischen der Syngamie und der ersten Zytokinese.

5. Verfahren nach Anspruch 1, wobei der eine oder die mehreren Embryonen durch die Befruchtung von Oozyten *in vitro* hergestellt sind, und wahlweise wobei die Oozyten *in vitro* herangereift sind, und wahlweise wobei die Oozyten, die *in vitro* herangereift sind, mit Wachstumsfaktoren ergänzt sind.

6. Verfahren nach Anspruch 1, wobei der eine oder die mehreren Embryonen vor der Kultivierung nicht eingefroren wurden.

7. Verfahren nach Anspruch 1, wobei der eine oder die mehreren Embryonen vor der Kultivierung eingefroren wurden.

8. Verfahren nach Anspruch 1, wobei die Deselektierung eines Embryos mit schlechtem Entwicklungspotenzial automatisiert ist.

9. Verfahren nach Anspruch 1, wobei die Zeitrafferaufnahme Aufnahmen macht, die digital gespeichert werden.

10. Verfahren nach Anspruch 1, wobei die Zeitrafferaufnahme Dunkelfeld-Ausleuchtung einsetzt.

11. Verfahren nach Anspruch 1, wobei der eine oder die mehreren menschlichen Embryonen vor der Kultivierung unter Bedingungen, die für die Embryonenentwicklung ausreichend sind, in eine Kulturschale gegeben werden.

12. Verfahren nach Anspruch 11, wobei die Kulturschale eine Vielzahl von MicroWells umfasst, und wahlweise wobei die Kulturschale 1 bis 30 MicroWells umfasst.

13. Verfahren nach Anspruch 12, wobei einer oder mehrere menschliche Embryonen vor der Kultivierung unter Bedingungen, die für die Embryonenentwicklung ausreichend sind, in einen MicroWell gegeben werden.

14. Verfahren nach Anspruch 1, wobei das Messen an einer Bilderzeugungsstation stattfindet.

**Revendications**

1. Procédé pour désélectionner un ou plusieurs embryons humains ayant un médiocre potentiel de développement, comprenant :

(A) la culture in vitro d'un ou plusieurs embryons sous des conditions suffisantes pour le développement d'embryon ;
(B) l'imagerie à intervale desdits un ou plusieurs embryons pendant une période de temps suffisante pour mesurer au moins une division cellulaire ; et
(C) la désélection d'un embryon ayant un médiocre potentiel de développement quand l'embryon affiche un phénotype anormal au second clivage (AC2)

désélectionnant de ce fait un embryon ayant un médiocre potentiel de développement, dans lequel l'embryon ayant un médiocre potentiel de développement ne sera pas susceptible d'atteindre le stade de blastocyste ou de s'implanter avec succès dans l'utérus.

2. Procédé selon la revendication 1, comprenant la désélection d'un embryon quand l'embryon affiche un phénotype anormal au second clivage (AC2) et au premier clivage (AC1).

**3.** Procédé selon la revendication 1, dans lequel la désélection comprend le choix de ne pas implanter dans l'utérus l'embryon déterminé comme ayant un médiocre potentiel de développement.

**4.** Procédé selon la revendication 1, comprenant en outre la mesure d'un ou plusieurs paramètres cellulaires sélectionnés à partir du groupe constitué par :

(a) la durée de la première cytocinèse ;
(b) le temps entre les première et deuxième mitoses ;
(c) le temps entre les deuxième et troisième mitoses ;
(d) l'intervalle de temps entre la cytocinèse 1 et la cytocinèse 2 ;
(e) l'intervalle de temps entre la cytocinèse 2 et la cytocinèse 3 ;
(f) l'intervalle de temps entre la fécondation et le stade cellulaire 5 ;
(g) la durée du premier cycle cellulaire ; et
(h) l'intervalle de temps entre la syngamie et la première cytocinèse.

**5.** Procédé selon la revendication 1, dans lequel lesdits un ou plusieurs embryons sont produits par fécondation d'ovocytes in vitro et de manière facultative dans lequel lesdits ovocytes sont vieillis in vitro et de manière facultative lesdits ovocytes vieillis in vitro sont complémentés avec des facteurs de croissance.

**6.** Procédé selon la revendication 1, dans lequel lesdits un ou plusieurs embryons n'ont pas été congelés avant culture.

**7.** Procédé selon la revendication 1, dans lequel lesdits un ou plusieurs embryons ont été congelés avant culture.

**8.** Procédé selon la revendication 1, dans lequel la désélection d'un embryon ayant un médiocre potentiel de développement est automatisée.

**9.** Procédé selon la revendication 1, dans lequel ladite imagerie à intervalle acquiert des images qui sont stockées de façon numérique.

**10.** Procédé selon la revendication 1, dans lequel ladite imagerie à intervalle utilise un éclairage en fond noir.

**11.** Procédé selon la revendication 1, dans lequel lesdits un ou plusieurs embryons humains sont placés dans une boîte pour culture avant culture sous des conditions suffisantes pour le développement d'embryon.

**12.** Procédé selon la revendication 11, dans lequel ladite boîte pour culture comprend une pluralité de micropuits et de manière facultative
dans lequel ladite boîte pour culture comprend de 1 à environ 30 micropuits.

**13.** Procédé selon la revendication 12, dans lequel un ou plusieurs embryons humains sont placés à l'intérieur d'un micropuits avant culture sous des conditions suffisantes pour le développement d'embryon.

**14.** Procédé selon la revendication 1, dans lequel la mesure est effectuée au niveau d'une station d'imagerie.

Figure 1

| EMBRYO PHENOTYPE | DEFINITION | SCHEMATIC | PREVALENCE |
|---|---|---|---|

Abnormal Syngamy (AS) — Embryos with disordered PN movement within the cytoplasm, accompanied by delayed dispersion of the nuclear envelopes

Abnormal Syngamy (AS) N=649
- Without AS N=443 68.3%
- With AS N=163 25.1%
- Immeasurable N=43 6.6%

Abnormal First Cytokinesis (A1$^{cyt}$) — Embryos exhibiting oolemma ruffling, and possible pseudo furrows, before first cytokinesis

Abnormal First Cytokinesis (A1$^{cyt}$) N=639
- Without A1$^{cyt}$ N=443 69.3%
- With A1$^{cyt}$ N=196 30.7%

Abnormal Cleavage (AC) — More than 2 cells produced during a single cell division event

AC1 first cleavage yielding >2 blastomeres

AC2 daughter cell cleavage yielding >2 blastomeres

Abnormal Cleavage (AC) N=639
- Without AC N=524 82.0%
- With AC N=115 18.0%
  - AC1 N=53 8.3%
  - AC2 N=59 9.2%
  - AC1 and AC2 N=3 0.5%

Chaotic Cleavage — Disordered cleavage behavior by the 4-cell stage

Chaotic Cleavage N=639
- Without Chaotic Cleavage N=543 85.0%
- With Chaotic Cleavage N=96 15.0%

Figure 2

Abnormal
Syngamy
(**AS**)

Abnormal
First Cytokinesis
(**A1**$^{cyt}$)

**AC1** first cleavage yielding >2 blastomeres

Abnormal
Cleavage
(**AC**)

**AC2** daughter cell cleavage yielding >2 blastomeres

Chaotic
Cleavage

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61759607 A
- US 61783988 A
- US 61818127 A
- US 7963906 B
- US 8323177 B
- US 8337387 B
- WO 2012163363 A
- US 6610543 B
- US 6130086 A
- US 5837543 A
- US 5882928 A
- US 6281013 B
- US 6777233 B

- US 7037892 B
- US 7029913 B
- US 5843780 A
- US 6200806 B
- US 20090047263 A
- US 20090068742 A
- US 2011053537 W
- US 61785170 W
- US 61785179 W
- US 61785199 W
- US 61785216 W
- US 61770998 W
- US 61771000 B

**Non-patent literature cited in the description**

- **TAFT, R.E.** *Theriogenology,* 2008, vol. 69 (1), 10-16
- **BELL, C. E. et al.** *Mol. Hum. Reprod.,* 2008, vol. 14, 691-701
- **BRAUDE, P. et al.** *Nature,* 1988, vol. 332, 459-461
- **HAMATANI, T. et al.** *Proc. Natl. Acad. Sci.,* 2004, vol. 101, 10326-10331
- **DOBSON, T. et al.** *Human Molecular Genetics,* 2004, vol. 13 (14), 1461-1470
- **DOBSON, T. et al.** *Human Molecular Genetics,* 2004, vol. 13 (14), 1461-1470
- **RIENZI, L. et al.** *Reprod. Biomed. Online,* 2005, vol. 10, 669-681
- **ALIKANI, M. et al.** *Mol. Hum. Reprod.,* 2005, vol. 11, 335-344
- **KELTZ, M. D. et al.** *Fertil. Steril.,* 2006, vol. 86, 321-324
- **FRENCH, D. B. et al.** *Fertil. Steril.,* 2009
- **ZERNICKA-GOETZ, M.** *Development,* 2002, vol. 129, 815-829
- **WANG, Q. et al.** *Dev Cell.,* 2004, vol. 6, 133-144
- **ZEMICKA-GOETZ, M.** *Curr. Opin. Genet. Dev.,* 2006, vol. 16, 406-412
- **MTANGO, N. R. et al.** *Int. Rev. Cell. Mol. Biol.,* 2008, vol. 268, 223-290
- **RIJINDERS PM ; JANSEN CAM.** *Hum Reprod,* 1998, vol. 13, 2869-73

- **MILKI AA et al.** *Fertil Steril,* 2002, vol. 77, 1191-5
- **MILKI A et al.** *Fertil Steril,* 2000, vol. 73, 126-9
- **FRAGOULI E.** *Fertil Steril,* 21 June 2009
- **EL-TOUKHY T et al.** *Hum Reprod,* 2009, vol. 6, 20
- **VANNESTE E et al.** *Nat Med,* 2009, vol. 15, 577-83
- **MANIPALVIRATN S et al.** *Fertil Steril,* 2009, vol. 91, 305-15
- **MASTENBROEK S et al.** *N Engl J Med.,* 2007, vol. 357, 9-17
- **RUBIO et al.** Limited Implantation Success of Direct-Cleaved Human Zygotes: A Time-Lapse Study. *Fertil. Steril.,* 2012, vol. 98, 1458-63
- **CAMPBELL et al.** Modeling a Risk of Classification of Anneuploidy in Human Embryos Using Non-Invasive Morphokinetics. *Reprod. Bioemed. Online,* 2013
- **BASILE et al.** Type of Culture Media Does Not Affect Embryo Kinetics: A Time-Lapse Analysis of Sibling Oocytes. *Human Reprod.,* 2013, vol. 28 (3), 634-41
- **AZZARELLO et al.** The Impact of Procuclei Morphology and Dynamicity on Live Birth Outcome After Time-Lapse Culture. *Human Reprod.,* 2012, vol. 27 (9), 2649-57

- **LEMMEN et al.** Kinetic Markers of Human Embryo Quality Using Time-Lapse Recordings of IVF/ICSI-Fertilize Oocytes. *Reprod. Biomed. Online,* 2008, vol. 17 (3), 385-91
- **KATARI et al.** *Hum Mol Genet.,* 2009, vol. 18 (20), 3769-78
- **SEPÚLVEDA et al.** *Fertil Steril.,* 2009, vol. 91 (5), 1765-70
- **RACOWSKY, C. et al.** *Fertil Steril,* 2010, vol. 94 (3), 1152-3
- **VERNON, M. et al.** *Fertil Steril,* 2011, vol. 95 (8), 2761-3